(19) **Europäisches Patentamt / European Patent Office / Office européen des brevets**

(11) **EP 4 656 132 A1**

(12) **EUROPEAN PATENT APPLICATION**

(43) Date of publication:
**03.12.2025 Bulletin 2025/49**

(21) Application number: **25179085.3**

(22) Date of filing: **27.05.2025**

(51) International Patent Classification (IPC):
**A61B 5/0537** (2021.01)　　**A61B 5/145** (2006.01)
**A61B 5/1468** (2006.01)　　**A61B 5/00** (2006.01)
**A61M 5/14** (2006.01)　　**G16H 20/17** (2018.01)

(52) Cooperative Patent Classification (CPC):
**A61B 5/1468; A61B 5/4839; A61M 5/14248;**
**A61M 5/1723; G16H 20/17; G16H 40/63;**
A61B 5/7257; A61M 2005/1726; A61M 2205/50;
A61M 2205/52; A61M 2230/201

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **31.05.2024  US 202463654411 P**
**13.05.2025  US 202519206602**

(71) Applicant: **Medtronic MiniMed, Inc.**
**Northridge, CA 91325-1219 (US)**

(72) Inventors:
• **Tong, Davy T.**
**Northridge, 91325 (US)**

• **Kwa, Timothy C.**
**Northridge, 91325 (US)**
• **Zhang, Guangping**
**Northridge, 91325 (US)**
• **Romo-Anselmo, Evan M.**
**Northridge, 91325 (US)**
• **Fusselman, Hsi C.**
**Northridge, 91325 (US)**
• **Dang, Kiem H.**
**Northridge, 91325 (US)**
• **Chattaraj, Sarnath**
**Northridge, 91325 (US)**

(74) Representative: **Zimmermann & Partner**
**Patentanwälte mbB**
**P.O. Box 330 920**
**80069 München (DE)**

(54) **CONCURRENT MULTI-FREQUENCY IMPEDANCE MEASUREMENT**

(57) Systems and methods for concurrent multi-frequency impedance measurements to characterize a substance of interest (e.g., analyte concentration) are disclosed herein. Generally, in some variations a method for characterizing a substance includes generating an input signal having a first set of frequency components across a plurality of frequencies, exciting an electrode arrangement with the input signal wherein the electrode arrangement is in contact with the substance. receiving an output signal from the excited electrode arrangement, the output signal having a second set of frequency components across the plurality of frequencies, determining an impedance signature of the substance across the plurality of frequencies, based on the first and second sets of frequency components, and characterizing the substance based on the impedance signature.

210 — generating an input signal having a first set of frequency components across a plurality of frequencies

220 — exciting an electrode arrangement with the input signal

230 — receiving an output signal from the excited electrode arrangement, the output signal having a second set of frequency components across the plurality of frequencies

240 — determining an impedance signature of the substance across the plurality of frequencies, based on the first and second sets of frequency components

250 — characterizing the substance based on the impedance signature

**FIG. 2**

EP 4 656 132 A1

## Description

CROSS-REFERENCE TO RELATED APPLICATIONS

This application claims the benefit of priority to U.S. Provisional Application No. 63/654,411, filed May 31, 2024, and US application no. 19/206,602 filed May 13, 2025, which is incorporated herein by reference in its entirety.

TECHNICAL FIELD

The present technology relates to systems and methods for concurrent multi-frequency impedance measurement.

BACKGROUND

Electrochemical impedance measurements of a substance can provide useful information about the substance and have various applications in medical technologies. Impedance measurements can generally be performed by applying an input excitation signal to electrodes that are in contact with the substance and analyzing a resulting output signal from the electrodes. In some applications, the electrodes can be excited at various excitation frequencies via multiple scans, to obtain an impedance spectrum of the substance that characterizes the impedance of the substance across the various excitation frequencies. For example, continuous glucose monitoring devices can determine a user's glucose levels by measuring an impedance spectrum of the user's blood, interstitial fluid, other tissue or body fluids, or other fluid including glucose, since the impedance spectrum of the blood will vary according to glucose concentration, health of the tissue, and other factors.

However, current techniques for determining an impedance spectrum of a substance have several drawbacks. For example, a specialized analog front end (AFE) chip is typically used to sense impedance, but such AFE chips add costs and complexity to the system, and consume power, which can increase manufacturing costs and lead to challenges in low-power applications due to decreased battery life. Additionally, the footprint size for AFE chips can be relatively large, making device miniaturization difficult in instances in which device miniaturization is desirable.

Thus, there is a need for new and improved systems and methods for performing electrochemical impedance measurements.

SUMMARY

Described herein are systems and methods for performing electrochemical impedance measurements across a range of excitation frequencies in a manner that is fast, cost-effective, yet still accurate for purposes of characterizing impedance of a substance in various applications.

Generally, in some variations, a method for identifying concentration of an analyte, the method includes generating an input signal having a first set of frequency components across a plurality of frequencies, exciting an electrode arrangement with the input signal, wherein the electrode arrangement is in contact with the analyte, receiving an output signal from the excited electrode arrangement, the output signal having a second set of frequency components across the plurality of frequencies, determining an impedance signature of the analyte across the plurality of frequencies, based on the first and second sets of frequency components, and determining a concentration of the analyte based on the impedance signature. In some variations, determining concentration of the analyte can include determining compositional information of the analyte.

In some variations, the input signal is a non-sinusoidal wave, such as a square wave, a step function, or a triangle wave, though the input signal can be other suitable non-sinusoidal wave. Furthermore, the input signal can have an operating excitation voltage range of +1.23 V to -1.23 V, or an operating excitation voltage range of +600 mV to -600 mV, or an operating excitation voltage range of +400 mV to -400 mV.

In some variations, determining the impedance signature of the analyte includes determining an impedance spectrum of the analyte. For example, determining the impedance signature of the analyte can include decomposing the input signal into the first set of frequency components, decomposing the output signal into the second set of frequency components, and determining an impedance of the analyte at each of the plurality of frequencies based on a comparison of the first and second sets of frequency components. In some variations, decomposing the input signal includes applying a first Fourier transform to the input signal, and decomposing the output signal includes applying a second Fourier transform to the output signal. Furthermore, in some variations, determining the concentration of the analyte includes comparing the impedance signature to a lookup table stored in one or more memory devices.

In some variations, the electrode arrangement is in contact with a fluid path of a fluid including the analyte. In some variations, the electrode arrangement includes two or more electrodes. Furthermore, one or more of the electrodes in the electrode arrangement can include at least one of a catalyst or enzyme.

In some variations, the electrode arrangement is in an analyte delivery device. In some of these variations, the method can further include controlling delivery of the analyte to a user, based at least in part on the determined concentration of the analyte. Controlling delivery of the analyte can, for example, include determining a volume of fluid including the analyte to be delivered to the user, and delivering the determined volume of fluid to the user. Additionally or alternatively, controlling delivery of the analyte can include providing an alert communicating an indication of the determined concentration of the analyte.

In some variations, the electrode arrangement is in an analyte monitoring device. For example, the analyte can include insulin.

Generally, in some variations, a system for measuring concentration of an analyte includes an electrode arrangement in contact with a fluid including the analyte, a processor, and a memory device operably coupled to the processor and storing instructions that, when executed by the processor, cause the system to perform operations including generating an input signal having a first set of frequency components across a plurality of frequencies, exciting the electrode arrangement with the input signal, receiving an output signal from the excited electrode arrangement, the output signal having a second set of frequency components across the plurality of frequencies, determining an impedance signature of the analyte across the plurality of frequencies, based on the first and second sets of frequency components, and determining a concentration of the analyte based on the impedance signature. In some variations, determining concentration of the analyte can include determining compositional information of the analyte.

In some variations, the input signal is a non-sinusoidal wave, such as a square wave, a step function, or a triangle wave, though the input signal can be other suitable non-sinusoidal wave. Furthermore, the input signal can have an operating excitation voltage range of +1.23 V to -1.23 V, or an operating excitation voltage range of +600 mV to -600 mV, or an operating excitation voltage range of +400 mV to -400 mV.

In some variations, determining the impedance signature of the analyte includes determining an impedance spectrum of the analyte. For example, determining the impedance signature of the analyte can include decomposing the input signal into the first set of frequency components, decomposing the output signal into the second set of frequency components, and determining an impedance of the analyte at each of the plurality of frequencies based on a comparison of the first and second sets of frequency components. In some variations, decomposing the input signal includes applying a first Fourier transform to the input signal, and decomposing the output signal includes applying a second Fourier transform to the output signal. Furthermore, in some variations, determining the concentration of the analyte includes comparing the impedance signature to a lookup table stored in one or more memory devices.

In some variations, the electrode arrangement includes a plurality of electrodes comprising a working electrode and a counter electrode. Furthermore, in some variations the plurality of electrodes further includes a reference electrode. At least one electrode of the plurality of electrodes can include at least one of a catalyst or an enzyme.

In some variations, the system includes an analyte delivery device, such as an insulin pump (e.g., patch pump device, tethered pump device, insulin pen). In some variations, the system includes an analyte monitoring device, such as a continuous glucose monitoring device.

Generally, in some variations, a method for characterizing a substance, the method includes generating an input signal having a first set of frequency components across a plurality of frequencies, exciting an electrode arrangement with the input signal, wherein the electrode arrangement is in contact with the substance, receiving an output signal from the excited electrode arrangement, the output signal having a second set of frequency components across the plurality of frequencies, determining an impedance signature of the substance across the plurality of frequencies, based on the first and second sets of frequency components, and characterizing the substance based on the impedance signature.

In some variations, the input signal is a non-sinusoidal wave, such as a square wave, a step function, or a triangle wave, though the input signal can be other suitable non-sinusoidal wave. Furthermore, the input signal can have an operating excitation voltage range of +1.23 V to -1.23 V, or an operating excitation voltage range of +600 mV to -600 mV, or an operating excitation voltage range of +400 mV to -400 mV.

In some variations, determining the impedance signature of the analyte comprises determining an impedance spectrum of the analyte. For example, determining the impedance signature of the analyte can include decomposing the input signal into the first set of frequency components, decomposing the output signal into the second set of frequency components, and determining an impedance of the analyte at each of the plurality of frequencies based on a comparison of the first and second sets of frequency components. In some variations, decomposing the input signal comprises applying a first Fourier transform to the input signal, and decomposing the output signal comprises applying a second Fourier transform to the output signal. Furthermore, in some variations, characterizing the substance includes comparing the impedance signature to a lookup table stored in one or more memory devices.

In some variations, the substance includes a medicament to be delivered to a patient, and the method can further include controlling delivery of the medicament to the patient based on the characterization of the substance.

Generally, in some variations, a system for characterizing a substance includes an electrode arrangement in contact with the substance, a processor, and a memory device operably coupled to the processor and storing instructions that, when executed by the processor, cause the system to perform operations including generating an input signal having a first set of frequency components across a plurality of frequencies, exciting the electrode arrangement with the input signal, wherein the electrode arrangement is in contact with the substance, receiving an output signal from the excited electrode arrangement, the output signal having a second set of frequency components across the plurality of frequencies, determining an impedance signature of the substance across the plurality of frequencies, based on the first and second sets of frequency components, and characterizing the substance based on the impedance signature.

In some variations, the input signal is a non-sinusoidal wave, such as a square wave, a step function, or a triangle wave, though the input signal can be other suitable non-sinusoidal wave. Furthermore, the input signal can have an operating excitation voltage range of +1.23 V to -1.23 V, or an operating excitation voltage range of +600 mV to -600 mV, or an operating excitation voltage range of +400 mV to -400 mV.

In some variations, determining the impedance signature of the analyte comprises determining an impedance spectrum of the analyte. For example, determining the impedance signature of the analyte can include decomposing the input signal into the first set of frequency components, decomposing the output signal into the second set of frequency components, and determining an impedance of the analyte at each of the plurality of frequencies based on a comparison of the first and second sets of frequency components. In some variations, decomposing the input signal comprises applying a first Fourier transform to the input signal, and decomposing the output signal comprises applying a second Fourier transform to the output signal. Furthermore, in some variations, characterizing the substance includes comparing the impedance signature to a lookup table stored in one or more memory devices.

In some variations, the substance comprises a medicament to be delivered to a patient, and the instructions, when executed by the processor, cause the system to perform operations comprising controlling delivery of the medicament to the patient based on the characterization of the substance.

Generally, in some variations, a method for configuring an insulin delivery system, the method includes generating an input signal having a first set of frequency components across a plurality of frequencies, exciting an electrode arrangement with the input signal, wherein the electrode arrangement is in contact with an insulin formulation in an infusion device, receiving an output signal from the excited electrode arrangement, the output signal having a second set of frequency components across the plurality of frequencies, determining an impedance signature of the insulin formulation across the plurality of frequencies, based on the first and second sets of frequency components, determining an insulin concentration of the insulin formulation based on the impedance signature, and configuring the infusion device based on the determined insulin concentration. For example, in some variations, configuring the infusion device includes preventing the infusion device from delivering the insulin formulation, setting delivery parameters based on the determined insulin concentration, and/or providing a prompt to a user to update delivery parameters.

In some variations, the infusion device includes a patch pump comprising a reservoir configured to hold the insulin formulation. In some of these variations, at least a portion of the electrode arrangement is located at an outlet of the reservoir, and/or at least a portion of the electrode arrangement is located in a body of the reservoir. Additionally or alternatively, in some variations the patch pump can include a cannula configured to infuse the insulin formulation into a user, and at least a portion of the electrode arrangement is located in a fluid flow path between the reservoir and the cannula.

In some variations, the infusion device includes a tethered infusion system comprising a reservoir configured to hold the insulin formulation. In some of these variations, at least a portion of the electrode arrangement is located at an outlet of the reservoir. Additionally or alternatively, in some variations, the tethered infusion system includes an infusion tubing fluidically coupled to the reservoir, and at least a portion of the electrode arrangement is located inline with the infusion tubing. Furthermore, in some variations the tethered infusion system can include an infusion hub fluidically coupled to the reservoir, and at least a portion of the electrode arrangement can be located in the infusion hub. Additionally or alternatively, at least a portion of the electrode arrangement is located in a body of the reservoir and/or in a stopper of the reservoir.

In some variations, the infusion device comprises an infusion pen comprising a reservoir configured to hold the insulin formulation. In some of these variations, at least a portion of the electrode arrangement is located in a body of the reservoir and/or in a stopper of the reservoir.

Generally, in some variations, an insulin delivery system includes an infusion device comprising a reservoir configured to receive an insulin formulation, an electrode arrangement in contact with the insulin formulation, a processor, and a memory device operably coupled to the processor and storing instructions that, when executed by the processor, cause the insulin delivery system to perform operations including generating an input signal having a first set of frequency components across a plurality of frequencies, exciting the electrode arrangement with the input signal, wherein the electrode arrangement is in contact with the insulin formulation, receiving an output signal from the excited electrode arrangement, the output signal having a second set of frequency components across the plurality of frequencies, determining an impedance signature of the insulin formulation across the plurality of frequencies, based on the first and second sets of frequency components, characterizing the insulin formulation based on the impedance signature, and configuring the infusion device based on the characterization of the insulin formulation.

For example, in some variations, configuring the infusion device includes preventing the infusion device from delivering the insulin formulation, setting delivery parameters based on the determined insulin concentration, and/or providing a prompt to a user to update delivery parameters.

In some variations, the infusion device includes a patch pump comprising a reservoir configured to hold the insulin formulation. In some of these variations, at least a portion of the electrode arrangement is located at an outlet of the reservoir, and/or at least a portion of the electrode arrangement is located in a body of the reservoir. Additionally or alternatively, in some variations the patch pump can include a cannula configured to infuse the insulin formulation into a user, and at least a portion of the electrode arrangement is located in a fluid flow path between the reservoir and the cannula.

In some variations, the infusion device includes a tethered infusion system comprising a reservoir configured to hold the insulin formulation. In some of these variations, at least a portion of the electrode arrangement is located at an outlet of the reservoir. Additionally or alternatively, in some variations, the tethered infusion system includes an infusion tubing fluidically coupled to the reservoir, and at least a portion of the electrode arrangement is located inline with the infusion tubing. Furthermore, in some variations the tethered infusion system can include an infusion hub fluidically coupled to the reservoir, and at least a portion of the electrode arrangement can be located in the infusion hub. Additionally or alternatively, at least a portion of the electrode arrangement is located in a body of the reservoir and/or in a stopper of the reservoir.

In some variations, the infusion device comprises an infusion pen comprising a reservoir configured to hold the insulin formulation. In some of these variations, at least a portion of the electrode arrangement is located in a body of the reservoir and/or in a stopper of the reservoir.

Further disclosed herein are systems and methods for concurrent multi-frequency impedance measurements to characterize a substance of interest (e.g., analyte concentration). Generally, in some variations a method for characterizing a substance includes generating an input signal having a first set of frequency components across a plurality of frequencies, exciting an electrode arrangement with the input signal wherein the electrode arrangement is in contact with the substance. receiving an output signal from the excited electrode arrangement, the output signal having a second set of frequency components across the plurality of frequencies, determining an impedance signature of the substance across the plurality of frequencies, based on the first and second sets of frequency components, and characterizing the substance based on the impedance signature.

BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.

FIGS. 1A and 1B are illustrative schematics of example impedance measurement systems, in accordance with the present technology.

FIG. 2 is a flowchart schematic of an example method for performing concurrent multi-frequency impedance measurements to characterize a substance, in accordance with the present technology.

FIG. 3 is an illustrative schematic of an example method for performing concurrent multi-frequency impedance measurements, in accordance with the present technology.

FIG. 4 is an illustrative schematic of decomposition of an example signal in the time domain into frequency components by application of a Fourier transform.

FIG. 5 is a flowchart schematic of an example method for performing concurrent multi-frequency impedance measurements to determine a concentration of an analyte, in accordance with the present technology.

FIG. 6 illustrates example output signals in the time domain provided by an electrode arrangement excited while in contact with various insulin formulations of different insulin concentrations.

FIG. 7 illustrates example impedance signatures for various insulin formulations of different insulin concentrations when using a square wave as an input signal.

FIGS. 8-10 are schematic illustrations of example patch infusion devices with an electrode arrangement, in accordance with the present technology.

FIG. 11 is a schematic illustration of a tethered infusion device, in accordance with the present technology.

FIGS. 12-15 are schematic illustrations of portions of example tethered infusion devices with an electrode arrangement, in accordance with the present technology.

FIGS. 16A and 16B are assembled and exploded schematic views, respectively, of an infusion pen device with an electrode arrangement, in accordance with the present technology.

FIGS. 17A and 17B are exploded and detailed schematic views, respectively, of a transfer guard with an electrode arrangement, in accordance with the present technology.

DETAILED DESCRIPTION

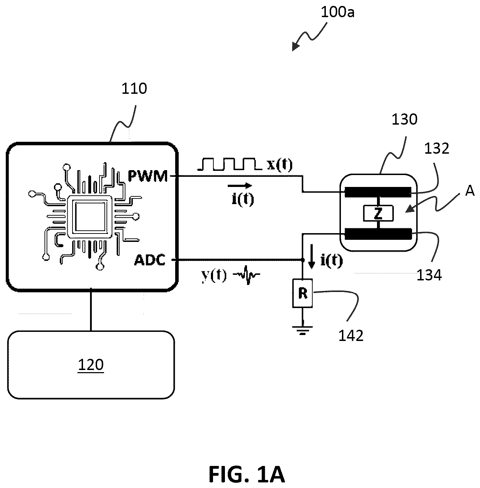

The present technology relates to systems and methods for concurrent multi-frequency impedance measurements. Some variations of the present technology, for example, are directed to determination of concentration of an analyte (e.g., insulin or other active component in a medicament formulation, or an excipient in a medicament formulation) in a fluid based on an impedance spectrum of the fluid. Specific details of several embodiments of the technology are described below with reference to FIGS. 1A-16B.

I. Concurrent multi-frequency impedance measurement

Described herein are system and methods for performing concurrent multi-frequency impedance measurements, such as for characterizing a substance. Concurrent multi-frequency impedance measurements can be performed by a suitable impedance measurement system including a processor, an electrode arrangement in contact with the substance to be characterized, and associated circuitry for signal processing. In some variations, the systems and methods in accordance with the present technology can be used to perform impedance measurements such as when a medical device including the electrode arrangement is in use (or is being prepared for use) by a patient. However, in some variations the systems and methods described herein can additionally or alternatively be used to characterize a subject in any suitable analytical setting (e.g., benchtop testing). Advantageously, the concurrent multi-frequency impedance measurement can be performed with a standard microprocessor such as a microprocessor that includes pulse width modulator (PWM) and analog-to-digital converter (ADC) functions. In other words, the concurrent multi-frequency impedance measurement advantageously does do not require a specialized AFE chip in order to measure impedance of a substance. In many applications that already require use of a microprocessor or microcontroller, eliminating the need for an AFE chip can reduce device costs, reduce power consumption, extend battery life, and/or reduce device size, among other benefits.

Furthermore, in contrast to how an AFE chip must perform multiple scans of the substance to obtain an impedance spectrum (that is, apply an individual input excitation signal to the electrode arrangement for each frequency separately and in sequence, which can be excessively power- and time-consuming), the concurrent multi-frequency impedance measurement techniques such as that described herein can obtain an impedance spectrum much more efficiently in a single scan. The single scan reduces power and time requirements in a device, resulting in additional benefits as well. For example, in a monitoring device (e.g., patient monitoring device such as a continuous glucose monitoring device), the ability to perform concurrent multi-frequency impedance measurements in a power- and time-efficient manner can enable more frequent impedance measurements, which can improve resolution of monitoring and patient outcomes (e.g., enable more prompt corrective actions for treatment, etc.). Furthermore, in certain medical device applications where impedance is used to monitor tissue health, the time-efficient nature of the present invention can mean the difference between life or death of the patient.

A. Impedance measurement systems

FIG. 1A is a simplified schematic illustration of an example impedance measurement system 100a, which includes at least one processor 110 (e.g., microcontroller), at least one memory device 120, and an electrode arrangement 130. In some variations, a separate memory device 120 may not be present (e.g., the processor can include a memory device). The processor 110 may be configured to execute the instructions that are stored in the memory device 120 such that, when it executes the instructions, the processor 110 performs aspects of the methods described herein. The instructions may be executed by computer-executable components integrated with a software application, applet, host, server, network, website, communication service, communication interface, hardware, firmware, software elements of a user computer or mobile device, smartphone, or any suitable combination thereof. In some variations, the processor 110 can include a pulse width modulator (PWM) and an analog-to-digital converter (ADC). The PWM can be configured to generate a suitable input excitation signal to be applied to the electrode arrangement 130 as further described below. However, in some variations the processor 110 may lack a PWM, and instead be configured to generate a suitable input signal composed of 1's and 0's, for example (e.g., "bit banging"). As described in further detail herein, the input signal generated by the processor 110 can have a set of multiple frequency components across a plurality of frequencies. The ADC can be configured to receive an analog output signal from the electrode arrangement 130 and convert the analog output signal to a digital signal for analysis (e.g., interpretation into one or more impedance measurements), as further described below.

The electrode arrangement 130 can include a plurality of electrodes and can be configured to be placed in contact with the substance whose impedance is to be measured. Collectively, the electrode arrangement and the substance of interest can form an electrochemical system with a characteristic impedance reflective of the impedance of the substance of interest. As shown in FIG. 1A, for example, the electrode arrangement 130 can include at least a working electrode 132 and a counter electrode 134. In some variations, the electrode arrangement 130 can include a working electrode 132 and a reference electrode in place of the counter electrode 134. In some variations, the electrode arrangement 130 can include at least three electrodes, including a working electrode 132, a counter electrode, and a reference electrode. The plurality of electrodes can include any suitable conductive material (e.g., platinum, gold), and be any suitable shape and size.

In some variations, at least one of the electrodes (e.g., working electrode 132) in the electrode arrangement 130 can include a layer with a catalyst, an enzyme, and/or other suitable mediator configured to lower the reaction energy for a certain desired reaction and increase the occurrence of the chemical reaction in the electrochemical system with the electrode arrangement 130, for the purposes of enhancing selectivity or increasing signal-to-noise ratio. Suitable catalysts can, for example, amplify the measured impedance differences among various substances. For example, in variations in which the impedance measurements are used to characterize insulin concentration in an insulin formulation, the insulin-sensing capability can be amplified by adding dithizone, insulin binding substances or proteins, insulinase, NiO, molecular carbon nanotubes, and/or other suitable cation or anion substance to one or more of the electrodes in the electrode arrangement 130. Such a mediator can amplify the differences in measured impedance differences among various insulin concentrations, which can in some instances result in the quantitative grouping of impedance signatures for similar insulin concentrations while reducing the impact of variance among manufacturers for similar insulin concentrations. For example, the addition of a suitable mediator in at least the working electrode can result in the quantitative grouping of impedance signatures for all U100 insulin formulations among various manufacturers, the quantitative grouping of impedance signatures for all U200 insulin formulations among various manufacturers, the quantitative grouping of impedance signatures for all U500 insulin formulations among various manufacturers, etc. Accordingly, such mediator(s) (e.g., catalyst, enzyme, etc.) may help enable characterizations of insulin concentrations that are more manufacturer-agnostic.

As another example, in variations in which the impedance measurements are used to measure glucose levels (e.g., in a continuous glucose monitor), at least one of the electrodes (e.g., working electrode) can include at least one mediator including the enzyme glucose oxidase and/or a catalyst such as Ruthenium-based catalysts (e.g. Ruthenium/-Carbon, Ruthenium/Nickel, Ruthenium/HY Zeolite, or Ruthenium/NiO/TiO2), an anti-insulin antibody, a metal chelator, an osmium-containing mediator, and/or other mediator that enhances the chemical reaction involving the oxidation of glucose oxidase. Such mediators are catalysts that can increase the reaction responsible for sensing glucose (e.g., increasing the signal of interest and/or decrease noise around the signal of interest), thereby amplifying the glucose-sensing capability of the impedance measurement system. However, in some variations the electrodes in the electrode arrangement 130 can include glucose oxidase while omitting a catalyst. In other variations, the electrode arrangement 130 can include a catalyst and omit glucose oxidase. Similarly in general, in some variations, the electrode arrangement 130 can include a sensing enzyme with a catalyst, while in some variations the electrode arrangement 130 can include an enzyme without a catalyst, or a catalyst without an enzyme.

Additionally or alternatively, at least one of the electrodes in the electrode arrangement 130 can include a mediator to improve the efficiency of a chemical reaction enabling sensing. For example, in variations in which the impedance measurements are used to measure glucose levels (e.g., in a continuous glucose monitor), at least one of the electrodes (e.g., working electrode) can include the enzyme glucose oxidase and a redox mediator such as ferrocene derivatives, ferricyanide, quinone compounds, conducting polymer salt tetrathiafulvalene-tetracyanoquinodimethane (TTF-TCNQ), transition metal complexes (e.g., ruthenium complexes), and phenothiazine. The addition of a redox mediator to the reaction can facilitate the efficient transfer of electrons between the redox center of glucose oxidase enzyme and the electrode. However, in some variations the electrodes in the electrode arrangement 130 can include glucose oxidase while omitting the mediator. Thus, in some variations, the electrode arrangement 130 can include a sensing enzyme with a mediator, while in some variations the electrode arrangement 130 can include a sensing enzyme without a mediator.

As shown in FIG. 1A, the plurality of electrodes can be positioned in contact with the substance whose impedance Z is to be measured, such as an analyte A (e.g., a fluid with an analyte). For example, the analyte A can include insulin, and it may be desired to identify an insulin formulation in order to ascertain the insulin concentration of the insulin formulation, such as in an insulin pump. More detailed examples of medicament delivery devices (e.g., insulin pumps) with an electrode arrangement 130 for concurrent multi-frequency impedance measurements are described in further detail below. As another example, the analyte A can include glucose, and it may be desired to measure the glucose level in a sample of patient fluid (e.g., interstitial fluid, blood), such as in a glucose monitoring device. However, the analyte A can include any suitable analyte whose concentration is desired to be measured.

Other examples of substances that can characterized include: target tissue that is intended to be in contact with an electrode where it may be desired to monitor the integrity of an electrode-tissue interface (e.g., for neurostimulation, neurorecording, tissue ablation, etc.), target tissue that is desired to be monitored for detecting disease state, and target tissue whose composition is desired to be characterized.

Generally, the processor 110 can be configured to generate (e.g., via the PWM) the input signal x(t), which can be applied to the electrode arrangement 130 (e.g., at least the working electrode 132) to excite the plurality of electrodes. In response, the electrode arrangement 130 can generate an output signal y(t) that is representative of the impedance Z of the analyte A. The output signal y(t) can be delivered to the processor 110, where the ADC converts the output signal y(t) to a digital signal for further analysis. FIG. 1A illustrates an example current path i(t) passing from the processor 110 to the electrode arrangement 130 to excite the electrode arrangement 130, and then passing from the electrode arrangement 130 through a pull-down resistor 142 to ground, with the output signal y(t) being sampled between the electrode arrangement 130 and the pull-down resistor 142. In some variations, the pull-down resistor 142 functions to enable the sampling of the output signal y(t) and can be selected to vary the sensitivity of the impedance measurement. In some variations, the resistance R of the pull-down resistor 142 can be selected to roughly correlate to (e.g., be generally equivalent to) the impedance of the substance to be characterized, which may help maximize the sensitivity of the impedance measurement or scan. For example, the resistance R can be within 10%, or within 5%, or within 3%, or within 2%, or within 1% of the average or expected impedance of the substance to be characterized. In one specific example in which insulin concentration of an insulin formulation is to be characterized by obtaining an impedance spectrum across a frequency range of about 1-32 kHz, the resistance R of the pull-down resistor 142 can be about 10 kOhms, which correlates to an average expected impedance of insulin formulations that may be measured (e.g., U100, U200, and U500).

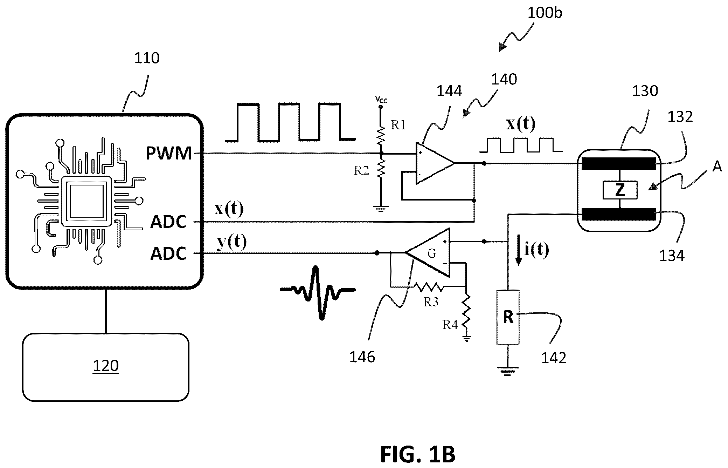

In some variations, the impedance measurement system can include additional circuitry, such as for signal processing of the input signal x(t) for exciting the electrode arrangement, and/or signal processing of the output signal y(t) received from the excited electrode arrangement. For example, FIG. 1B is a schematic illustration of a more detailed example impedance measurement system 100a, which is similar to the impedance measurement system 100a described above with respect to FIG. 1A except as described below. The processor 110, memory 120, electrode arrangement 130, and pull-down resistor 142 of impedance measurement system 100b can be similar to the processor 110, memory 120, electrode arrangement 130, and pull-down resistor 142 of impedance measurement system 100a. In the impedance measurement system 100b, the processor 110 can include two ADCs, one for receiving the input signal x(t) and another for receiving the output signal y(t), as further described below.

The impedance measurement system 100b can further include circuitry 140 including an input op-amp 144 (and associated resistors R1 and R2) and an output op-amp 146 (and associated resistors R3 and R4). In some variations such as that shown in FIG. 1B, the input op-amp 144 is an attenuating op-amp that functions to attenuate a raw signal from the PWM down to a suitable voltage range, thereby transforming the raw signal into an input signal x(t) having a suitable voltage level for exciting the electrode arrangement 130. For analytes that react above certain threshold voltages, it can be advantageous to attenuate the input signal in such a manner. Accordingly, the input signal x(t) as an output of the input op-amp 144 can be delivered to the electrode arrangement 130 (e.g., at least the working electrode 132), as well as sampled and delivered back to a first ADC. Furthermore, the output op-amp 146 can be an amplifying op-amp that functions to amplify the output signal from the electrode arrangement 130 (e.g., the counter electrode 142) up to a suitable voltage range for the second ADC, thereby transforming the output signal into a suitable voltage level for conversion to a digital signal. In some example variations, the input op-amp 144 (and/or associated resistors R1 and R2) can be configured to attenuate the input signal to a maximum voltage magnitude of about 250 mV, or to a maximum voltage magnitude of about 600 mV, or to a maximum voltage magnitude of about 1.23V. In some example variations, the output op-amp 146 (and/or associated resistors R3 and R4) can be configured with suitable gain G to amplify the output signal (e.g., to a maximum voltage magnitude of about 5 V). However, in some variations the input op-amp 144 is not an attenuating op-amp, and/or the output op-amp 146 is not an amplifying op-amp. Furthermore, in some variations, the input op-amp 144 can be an amplifying op-amp, and/or the output op-amp 146 can be an attenuating op-amp.

Further operation of an impedance measurement system (e.g., impedance measurement systems 100a or 100b) for performing concurrent multi-frequency impedance measurements is described in further detail below.

B. Impedance measurement methods

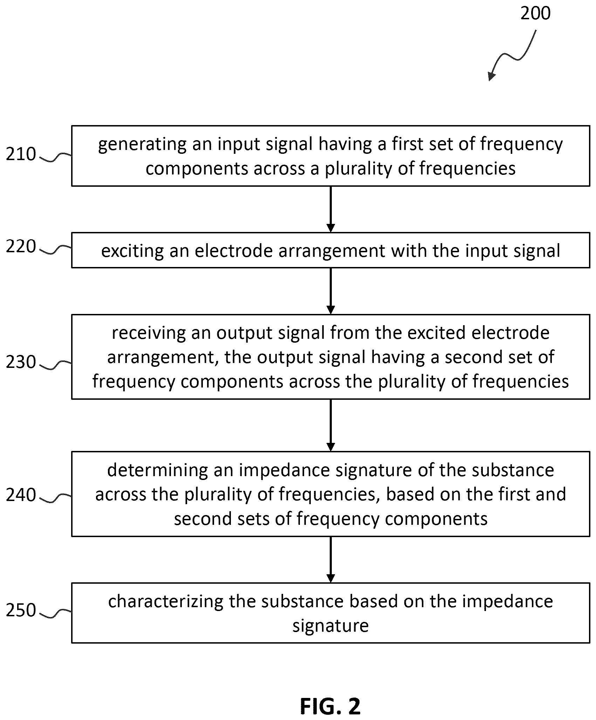

FIG. 2 illustrates a flowchart schematic of an example method 200 for performing concurrent multi-frequency impedance measurements, such as for characterizing a substance. As shown in FIG. 2, the method 200 can include generating an input signal 210 having a first set of frequency components across a plurality of frequencies, exciting an electrode arrangement 220 with the input signal where the electrode arrangement is in contact with the substance to be characterized, receiving an output signal from the excited electrode arrangement 230 where the output signal has a second set of frequency components across the plurality of frequencies, determining an impedance signature 240 of the substance across the plurality of frequencies based on the first and second sets of frequency components, and characterizing the substance 250 based on the impedance signature.

Generating an input signal 210 functions to produce an excitation signal for exciting the electrode arrangement (e.g., electrode arrangement 130 as described above with respect to FIGS. 1A and 1B) of an impedance measurement system. The input signal can have a set of multiple frequency components across a plurality of frequencies. In some variations, the input signal can be non-sinusoidal. For example, the input signal can be a square wave, which comprises multiple sine waves at various frequencies that are superimposed on each other. That is, a square wave is an example of a signal comprising a plurality of frequencies. The square wave can, for example, be generated with a PWM of a processor (e.g., processor 110) that generates variable width square wave pulses (e.g., with 25% duty cycle, 50% duty cycle, 75% duty, etc.). Other examples of an input signal are a step function or a triangle wave, though the input signal can include any suitable non-sinusoidal wave. The input signal can be designed such that its frequency components span across a plurality of frequencies suitable for characterizing the substance of interest. Suitable frequencies may be determined empirically, for example. As one example in which insulin concentration of an insulin formulation is desired to be determined through the impedance analysis, the input signal can have frequency components across a plurality of frequencies between about 0 kHz and about 32 kHz, or between about 0 kHz and about 12 kHz. However, the frequencies may vary depending on the nature of the substance to be characterized.

As described above, a signal generated by a processor of the impedance measurement system (e.g., processor 110) can be transformed into an input signal of suitable voltage range for excitation of the electrode arrangement (e.g., by circuitry 140). For example, the input signal that is delivered to the electrode arrangement can range between -1.23 V and +1.23 V (corresponding to the electrolysis of water, in view of applications in which the substance of interest is, or is included in, an aqueous solution), or between -600 mV and +600 mV, or between -400 mV and +400 mV, or between -250 mV and +250 mV. In some variations, for example, an input signal voltage range of between -250 mV and +250 mV has been found to help reduce the possibility of inadvertently exciting preservatives that are typically in insulin formulations, as exciting preservatives may lead to signal interference or noise in the output signal otherwise characterizing the insulin concentration in the insulin formulation.

Exciting the electrode arrangement 220 with the input signal induces the electrode arrangement to produce an output signal corresponding to the impedance of the substance to be characterized. As described above, the electrode arrangement (e.g., electrode arrangement 130) can include a plurality of electrodes, including a working electrode, a counter electrode, and/or a reference electrode. The plurality of electrodes, when excited by the input signal, can be in contact with the substance to be characterized. For example, the electrodes can be in contact with a fluid including an analyte of interest, in contact with a tissue interface, and/or the like. Further examples of electrode configurations are described elsewhere herein. The input signal, having multiple frequency components across multiple frequencies, is configured to excite the electrode arrangement at multiple frequencies concurrently.

Once excited when in contact with the substance to be characterized, the electrode arrangement produces an output signal that reflects the impedance of the substance. Receiving the output signal 230 functions to receive the output signal for analysis. Like the input signal, the output signal can have a set of multiple frequency components across a plurality of frequencies (which can, for example, be the same plurality of frequencies associated with the input signal). In some variations, the output signal can be processed before and/or after being received by the processor (e.g., processor 110). In some variations, the output signal can be amplified (e.g., by circuitry 140) to increase its voltage level to a level suitable for the ADC of the processor. For example, in some variations the output signal delivered to the ADC can be amplified with suitable gain to range between about -5 V and about +5 V.

Additionally or alternatively, the output signal can be processed to remove noise. For example, in some variations the output signal can be processed with a suitable low pass filter to filter out any unwanted harmonics and/or other sources of noise. One or more signal smoothing algorithms can additionally or alternatively be applied to reduce noise in the output signal. Additionally or alternatively, a suitable filter (e.g., band-pass and/or high-pass filter, etc.) can be implemented to pass through only those frequency components of interest in the output signal, which may vary depending on the application. As one example in which insulin concentration of an insulin formulation is desired to be determined through the impedance analysis, the output signal can be filtered with a suitable filter to pass through only those frequency components between about 0 kHz and about 32 kHz, or between about 0 kHz and about 12 kHz. However, the frequencies may vary depending on the nature of the substance to be characterized.

Determining the impedance signature 240 of the substance functions to generate impedance data for characterizing the substance. In some variations, the impedance signature comprises an impedance spectrum of the substance across the plurality of frequencies associated with the input and output signals. The impedance signature can be based at least in part on the set of frequency components of the input signal, and the set of frequency components of the output signal. For example, the impedance magnitude of the substance of interest at each frequency (that is, each frequency among the frequency components of the input and output signals) can be calculated using Ohm's law.

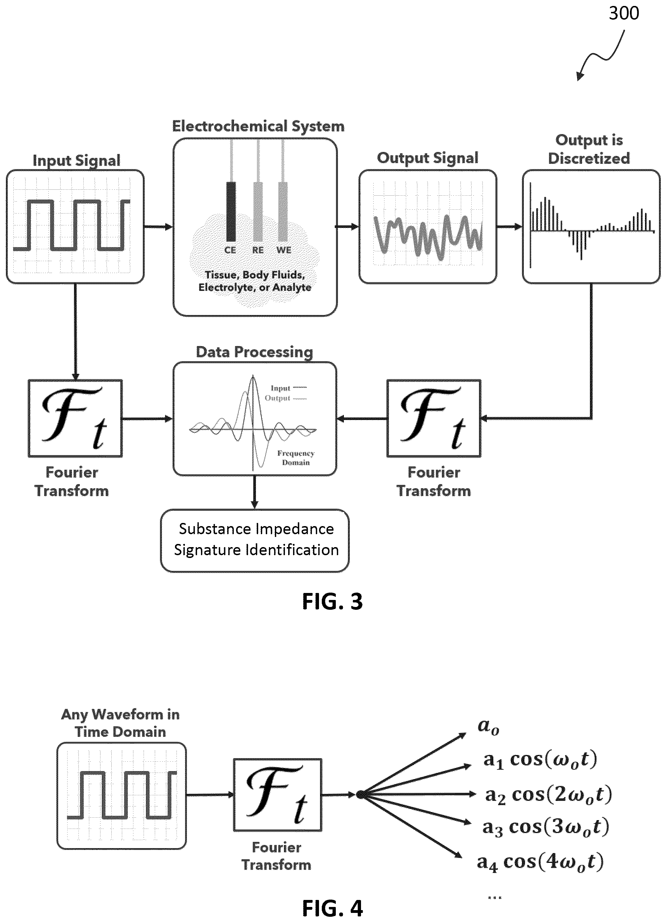

In some variations, determining the impedance signature comprises decomposing the input signal into its set of frequency components, decomposing the output signal into its set of frequency components, and determining an impedance of the analyte at each of the plurality of frequencies based on a comparison of the sets of frequency components for the input and output signals. FIG. 3 illustrates schematically various aspects of the process of determining the impedance signature. As shown in FIG. 3, the input signal can be decomposed into its frequency components by application of a Fourier transform. A suitable Fourier transform (e.g., Fast Fourier Transform) can, as shown in FIG. 4, transform any waveform in the time domain into the frequency domain and break apart the time domain signal into its frequency components. Accordingly, a Fourier transform can decompose the input signal x(t) into frequency components as shown in Equation 1:

$$x(t) = a_o + a_1 \cos(\omega_o t) \ + \ a_2 \cos(2\omega_o t) + ... \qquad (1)$$

where the coefficients $a_0$, $a_1$, $a_2$, ... $a_k$ are voltage magnitudes at each discrete frequency.

Similarly, a Fourier transform can discretize and/or decompose the output signal y(t) into frequency components as shown in Equation 2:

$$y(t) = b_o + b_1 \cos(\omega_o t + \varphi_1) + b_2 \cos(\omega_o t + \varphi_2) + ... \qquad (2)$$

wherein the coefficients $b_0$, $b_1$, $b_2$, ... $b_k$ are voltage magnitudes at each discrete frequency. The output signal can be used in accordance with Equation 3 below, which is based on Ohm's law:

$$y(t) = x(t)\frac{R}{R+Z} \qquad (3)$$

where R is the resistance value of the pull-down resistor in the impedance measurement system (e.g., resistor 142), and Z is impedance of the substance of interest.

Impedance magnitude $|Z|_k$ of the substance of interest can be solved from Equation 3 as shown in Equation 4 for each discrete frequency. The calculation of Equation 4, when repeated across the various discrete frequencies, compares each frequency component of the input and output signals to acquire an impedance signature (e.g., impedance spectrum) of the substance of interest (e.g., analyte, tissue, body fluid, etc.) across the discrete frequencies.

$$|Z|_k = R\frac{a_k - b_k}{b_k} (4)$$

Furthermore, a phase $\phi$ of the output signal from the electrode arrangement can be calculated. In some variations, the substance being characterized (e.g., analyte) has a unique phase signature. Accordingly, the phase $\phi$ of the output signal can additionally or alternatively be used to characterize the substance. Additionally or alternatively, the phase $\phi$ can correlated to the function and performance of one or more electrodes of the electrode arrangement, as phase can be correlated to magnitude of the capacitive elements in the electrochemical system, with a large degree of frequency dependence. For example, changes to the capacitance in the electrochemical system can indicate changes in properties at the electrode surface (e.g., absorption, adsorption, polymerization, passivation, etc.), which can reflect function and/or performance of one or more electrodes in the electrochemical system.

The impedance signature can, in some variations, be represented as a curve that is the result of plotting the impedance magnitude $|Z|_k$ across the various discrete frequencies. The appearance of the impedance signature is characteristic of the substance of interest (e.g., analyte concentration, tissue health, etc.).

Characterizing the substance of interest 250 leverages the impedance signature to identify one or more aspects of the substance of interest. In some variations, characterizing the substance of interest can include comparing the impedance signature to a lookup table stored in one or more memory devices (e.g., memory 120). The lookup table can include various impedance signatures associated with different types of substances of interest, and can, for example, be generated empirically and stored in the memory device(s). Additionally or alternatively, the phase of the output signal can be leveraged to characterize the substance of interest (e.g., comparing the phase to a lookup table stored in one or more memory devices).

Various conclusions can be drawn based on the characterization of the substance of interest, depending on the application. For example, the impedance signature can be correlated or matched to a particular analyte concentration, a tissue disease state, nature, and/or integrity of an electrode-tissue interface, and/or the like.

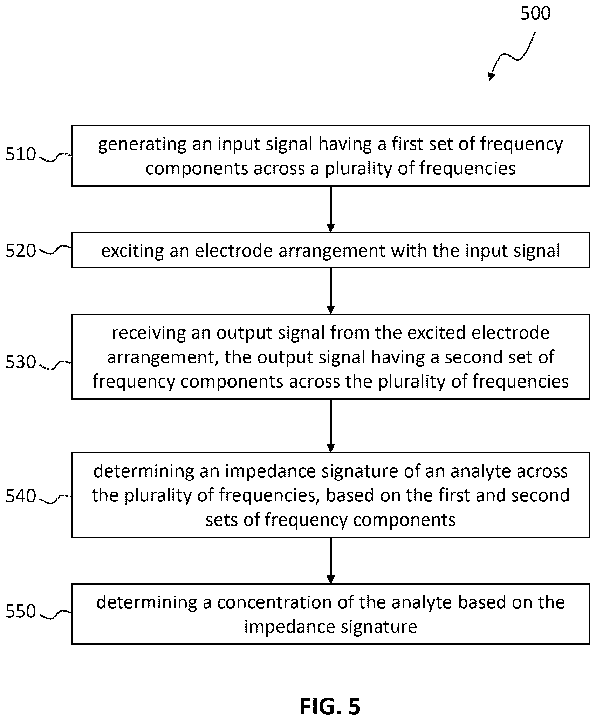

Accordingly, other variations of the method 200 can be performed for characterizing various kinds of substances and/or for different purposes. For example, as shown in FIG. 5, a method 500 is an example variation of method 200 in which the substance to be characterized is an analyte in a fluid. The method 500 can include generating an input signal 510 having a first set of frequency components across a plurality of frequencies, exciting an electrode arrangement 520 with the input signal where the electrode arrangement is in contact with an analyte to be characterized, receiving an output signal from the excited electrode arrangement 530 where the output signal has a second set of frequency components across the plurality of frequencies, determining an impedance signature 540 of the analyte across the plurality of frequencies based on the first and second sets of frequency components, and determining a concentration of the analyte based on the impedance signature 550. Many of these processes can be similar to the like-numbered processes in method 200 described above with respect to FIG. 2. For example, generating an input signal 510 can be similar to generating an input signal 210, exciting an electrode arrangement 520 can be similar to exciting an electrode arrangement 220, receiving an output signal 530 can be similar to receiving an output signal 230, and determining an impedance signature 540 can be similar to determining an impedance signature 240. Determining a concentration of the analyte 550 is a specific example of characterizing the substance of interest 250.

In some variations, the method 500 (or method 200) can be performed to identify the medicament (e.g., concentration of an analyte that is an active treatment component of the medicament) in a medicament delivery device. The method 500 can be performed to identify a concentration of the analyte (e.g., quantification of the analyte, specific type of formulation of the analyte, a specific composition of the analyte, etc.). For example, the method 500 (or method 200) can be performed to determine insulin concentration of an insulin formulation in an insulin pump device. This identification of insulin concentration can be useful, for example, to help ensure that a suitable volume of the insulin formulation is delivered to the patient to deliver a proper dose of insulin. In some variations, this can enable the insulin pump device to include any one of a variety of insulin formulations while reducing the risk of inadvertently delivering an improper dose of insulin to the patient. For example, the identification of insulin concentration can provide a safety check in the event that a patient loads the wrong insulin formulation in the insulin pump device, and/or neglect to inform the insulin pump device which insulin formulation was loaded. Accordingly, it can be advantageous for an insulin pump device to incorporate a method for performing impedance-based characterization of the insulin formulation, and as described herein, incorporating a method for concurrent multi-frequency impedance measurement can be further advantageous for reducing power consumption, increasing battery life, etc.

In some variations, a medicament delivery device can perform further action(s) based on the identification of the medicament. For example, a medicament delivery device (e.g., insulin pump device), once having identified a concentration of the medicament, can control delivery of the medicament accordingly. In some variations, controlling delivery of the medicament can include adjusting suitable dosage settings (e.g., a volume of the medicament to be delivered, ratios for purposes of determining volume of the medicament to be delivered, timing for delivery of the medicament, etc.). Additionally or alternatively, controlling delivery of the medicament can include providing an alert communicating an indication of the determined concentration of the medicament. The alert can, for example, communicate that an expected type of medicament has been correctly loaded into the medicament delivery device, communicate that an unexpected type of medicament has been incorrectly loaded into the medicament delivery device, and/or communicate the specific type of medicament itself that has been loaded into the medicament delivery device. Additionally or alternatively, controlling delivery of the medicament can include enabling or disabling a lockout feature for delivery of the medicament. For example, in response to determining that an expected type of medicament has been loaded into the medicament delivery device, the medicament delivery device can be enabled to provide delivery of the medicament. In contrast, in response to determining that an unexpected type of medicament has been loaded into the medicament delivery device, the medicament delivery device can be disabled to prevent delivery of the medicament (e.g., for patient safety purposes) and/or the medicament delivery device can prompt the user the adjust delivery parameters to correspond to the medicament loaded.

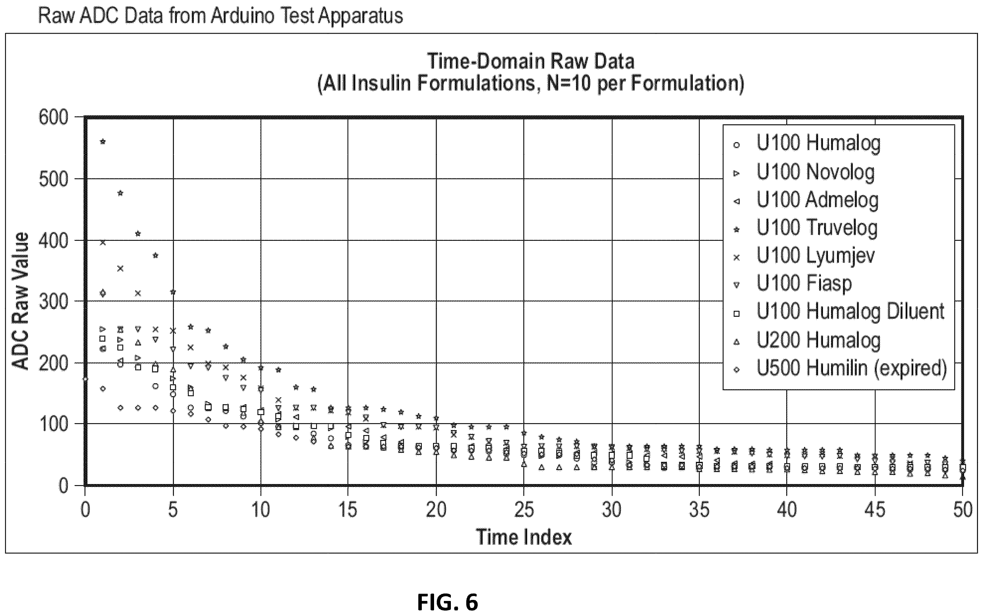

FIG. 6 is a plot of various example output signals in the time domain that are produced by an electrode arrangement excited with a square input wave in accordance with the present technology. The various example output signals are associated with different insulin concentrations in various insulin formulations in contact with the excited electrode arrangements. For each of ten instances (n=10) of testing on a particular insulin formulation (i.e., particular insulin concentration of U100, U200, or U500 and/or particular manufacturer), an output signal was produced by the excited electrode arrangement. The output signal data for the ten instances was averaged for each insulin formulation and plotted as shown in FIG. 6. As shown in FIG. 6, it is difficult to distinguish between at least some of the different insulin formulations based on the time domain output signals.

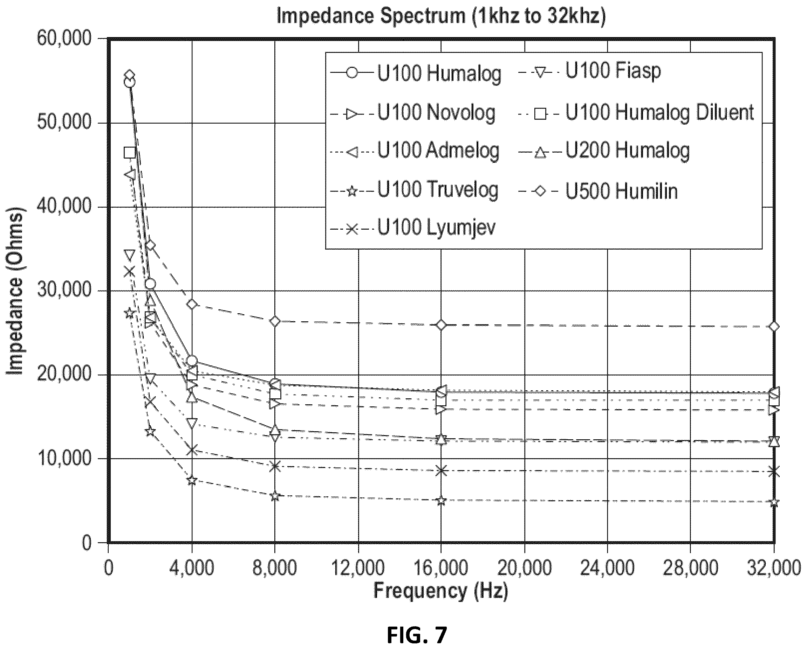

FIG. 7 is a plot of impedance spectrums of the various insulin formulations whose time domain output signals are shown in FIG. 6. The impedance spectrums were calculated as described above with respect to methods 200 and 500, across frequencies ranging from 1-32kHz. As shown in FIG. 7, the impedance spectrums for the various insulin formulations are more easily differentiated compared to the plot of FIG. 6. Thus, FIG. 7 indicates that the impedance spectrum of an insulin formulation, as obtained by concurrent multi-frequency impedance measurements, can be used to identify the concentration and/or manufacturer of insulin formulation that is in contact with an excited electrode arrangement, such as in an insulin pump delivery device.

As another example variation, a method for characterizing a substance with concurrent multi-frequency impedance measurements can include performing enzymatic biosensing. For example, the method can be performed to operate (e.g., read) a glucose sensor that includes electrodes in contact with a body fluid for glucose measurements, such as interstitial fluid, blood, etc. In some variations, enzymatic biosensing can be performed alone, or can be performed in combination with other impedance sensing (e.g., simultaneously). Enzymatic biosensors typically are operated by applying a constant applied DC bias voltage to the sensing electrode arrangement (e.g., to the working electrode). The applied DC bias voltage of the input signal can be represented by the initial term $a_o$ in the input signal decomposed by a Fourier transform per Equation 1 above. Thus, the DC component of an input square wave signal can be used to operate an enzymatic biosensor, and in some variations both glucose and impedance can be sensed simultaneously using the methods described herein.

## II. Example systems

In some variations, an impedance measurement system (e.g., impedance measurement system 100a or 100b) can be incorporated in a medical device. For example, an impedance measurement system can be incorporated in a medicament delivery device. The medicament delivery device can be configured to deliver a medicament including an analyte of interest whose concentration is desired to be measured. For example, an impedance measurement system can be incorporated in an infusion device or other insulin delivery system. Various examples of insulin delivery systems are described below.

## A. Patch pump

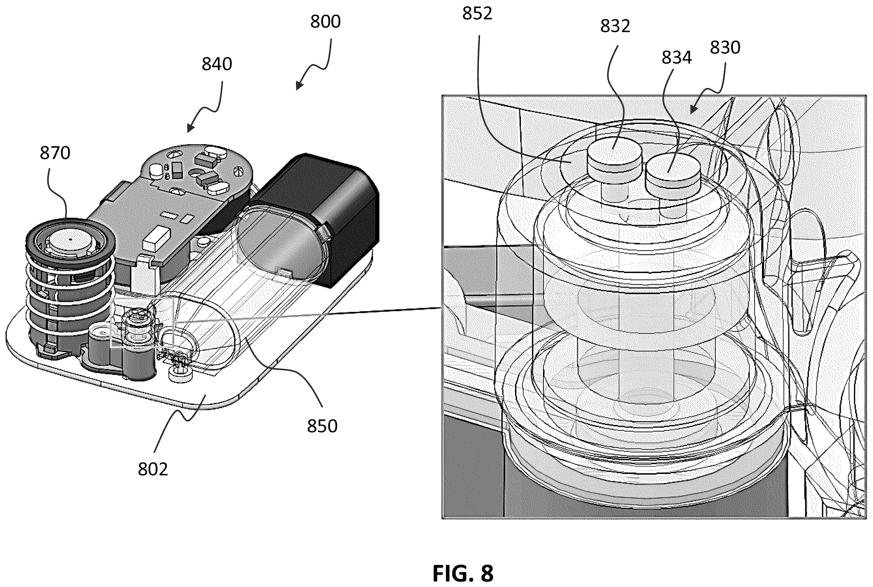

In some variations, an insulin delivery system includes an infusion device (also referred to herein as a patch pump) that is attachable as a wearable to a skin surface of a user. For example, FIG. 8 is a schematic illustration of an infusion device 800 that includes an adhesive 802 for attaching the infusion device 800 to a skin surface of a user. The infusion device 800 can include a housing (not shown, for clarity) that houses various components including a reservoir 850, an insertion assembly 870, and an electronics assembly 840 including circuitry for operating aspects of the infusion device 800. The reservoir 850 can be configured to hold an insulin formulation (or other suitable medicament) of any one of a range of insulin concentrations. In some variations, for example, the reservoir 850 can be filled with an insulin formulation having a high concentration of insulin (e.g., U200, U500) that can be delivered in smaller increments across more doses, thereby enabling an extended wear lifetime of the patch infusion device. For example, in some variations the patch infusion device can be configured for a wear lifetime of at least 5 days, at least 7 days, or at least 10 days. The reservoir 850 can be prefilled and/or user-fillable by way of a fill port (not shown). The insertion assembly 870 can include a cannula in fluidic communication with the reservoir 850 and insertable in a user for infusing the medicament into a user, a needle associated with the cannula to aid insertion of the cannula into the user, and one or more actuators for driving the needle and/or cannula into the user. The electronics assembly 840 can include a processor (e.g., processor 110), a memory device (e.g., memory device 120), and/or other suitable circuitry for controlling the infusion device 800 and/or performing one or more aspects of concurrent multi-frequency impedance measurements (e.g., in accordance with method 200 and/or method 500). Additionally or alternatively, a processor for performing one or more aspects of the concurrent multi-frequency impedance measurements can be located remotely from the electronics assembly 840. For example, an output signal from an excited electrode arrangement can be transmitted (e.g., in a wireless manner using Bluetooth or other suitable wireless communication protocol) to a separate processor not included in the infusion device 800, for analysis.

In some variations, the infusion device 800 can further include a sensor electrode configured to monitor one or more physiological parameters, such as glucose level. The sensor electrode can, for example, be configured to measure glucose levels via a concurrent multi-frequency impedance measurement as described herein, or in any other suitable manner. In other examples, the infusion device 800 can communicate with a sensor attached to the user external from the infusion device 800, such as an external constant glucose monitor. The infusion device 800 can, in some variations, be configured to deliver a suitable amount of insulin based on a glucose measurement for the user. Examples of attachment of an infusion device to a user and examples of use of the device are similar to that described in US Patent No. 10,159,786, assigned to Medtronic MiniMed, Inc., which is hereby incorporated by reference in its entirety.

However, it may be desirable to determine the insulin concentration of the insulin formulation that is in the reservoir 850, such as to avoid inadvertently administering the wrong dose of insulin to the user. For example, U200 insulin will require half the required dose of U100 in response to a specific glucose level. If the infusion device is set to dispense U100 insulin but instead is filled with U200 insulin, the user will be overdosed and may go hypoglycemic. Thus, knowing the correct insulin concentration within the reservoir is critical. Accordingly, the infusion device 800 can include an electrode arrangement (e.g., similar to electrode arrangement 130 described herein) that is configured to sense an impedance signature of the insulin formulation, and identify an insulin concentration based on the impedance signature. As shown in FIG. 8, the infusion device 800 can include an electrode arrangement 830 that may include a working electrode 832 and a counter electrode 834 that are arranged at an outlet 852 of the reservoir 850, where the working electrode 832 and the counter electrode 834 are configured to be in contact with the insulin formulation as it exits the reservoir 850. The electrode arrangement 830 is in electrical communication with the electronics assembly 840 such that the electrode arrangement 830 (e.g., at least the working electrode 832) can receive an input signal with multiple frequency components (e.g., as described above with respect to methods 200 and 500) that is generated by one or more processors (e.g., processor 110). Similar to that described above with respect to methods 200 and 500, the excited electrode arrangement 830 can produce an output signal that can be analyzed to determine an impedance signature of the insulin formulation. For example, the output signal can be indicative of the insulin formulation (e.g., concentration of insulin, such as U100, U200, U500, etc.). In some variations, the concurrent multi-frequency impedance measurement with the electrode arrangement 830 can be performed during a priming operation of the infusion device 800. Furthermore, in some variations the infusion device 800 can be configured to control delivery of the medicament based at least in part on the determination of the insulin formulation, similar to that described above with respect to method 500 (e.g., adjust dosage settings, providing an alert regarding the insulin concentration, enable or disable a lockout feature for delivery of the insulin formulation, etc.).

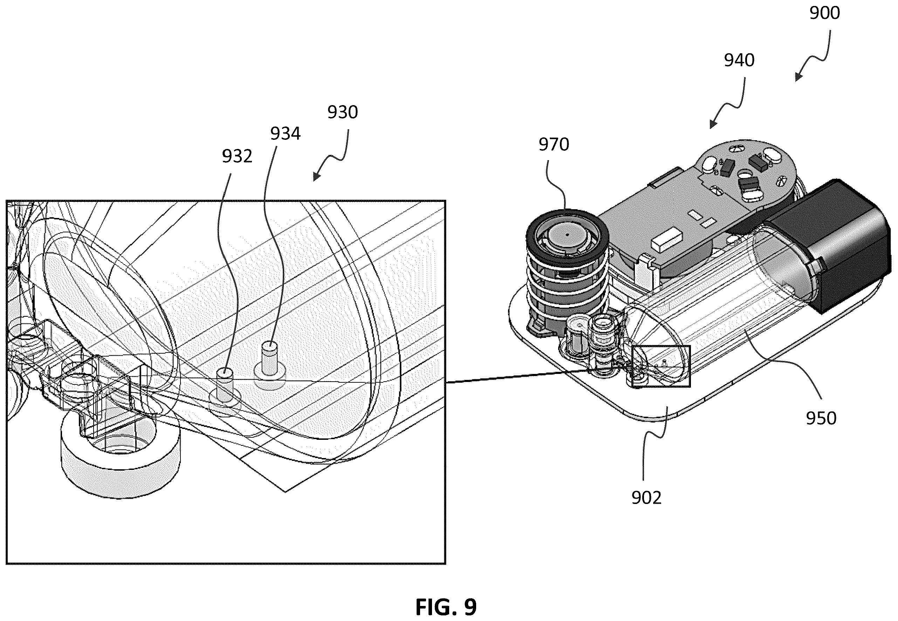

FIG. 9 is a schematic illustration of an example infusion device 900 that is similar to infusion device 800 described above with respect to FIG. 8 except as described below, and can include many components with like numbering as infusion device 800. For example, the infusion device 900 can include an adhesive 902 for attaching the infusion device 900 to a skin surface of a user. The infusion device 900 can include a housing that houses various components including a reservoir 950 (e.g., similar to reservoir 850), an insertion assembly 970 (e.g., similar to insertion assembly 870), and an electronics assembly 940 (e.g., similar to electronics assembly 840). However, infusion device 900 can include an electrode arrangement 930 that is located in the reservoir 950 itself, such as in or on a wall of the reservoir 950 such that the working electrode 932 and the counter electrode 934 of the electrode arrangement are in contact with the insulin formulation held in the reservoir 950. The electrode arrangement 930 is in electrical communication with the electronics assembly 940 such that the electrode arrangement 930 (e.g., at least the working electrode 932) can receive an input signal with multiple frequency components (e.g., as described above with respect to methods 200 and 500) that is generated by one or more processors (e.g., processor 110). Similar to that described above with respect to methods 200 and 500, the excited electrode arrangement 930 can produce an output signal that can be analyzed to determine an impedance signature of the insulin formulation. For example, the output signal can be indicative of the insulin formulation (e.g., concentration of insulin, such as U100, U200, U500, etc.). In some variations, the concurrent multi-frequency impedance measurement with the electrode arrangement 930 can be performed during a priming operation of the infusion device 900. Furthermore, in some variations the infusion device 900 can be configured to control delivery of the medicament based at least in part on the determination of the insulin formulation, similar to that described above with respect to method 500 (e.g., adjust dosage settings, providing an alert regarding the insulin concentration, enable or disable a lockout feature for delivery of the insulin formulation, etc.).

In some variations, the electrode arrangement 930 can additionally or alternatively be used to detect when the reservoir 950 is empty and/or holds an otherwise insufficient amount of insulin formulation. For example, the impedance signature detected by the electrode arrangement 930 can correspond to that of air (e.g., as pre-determined empirically).

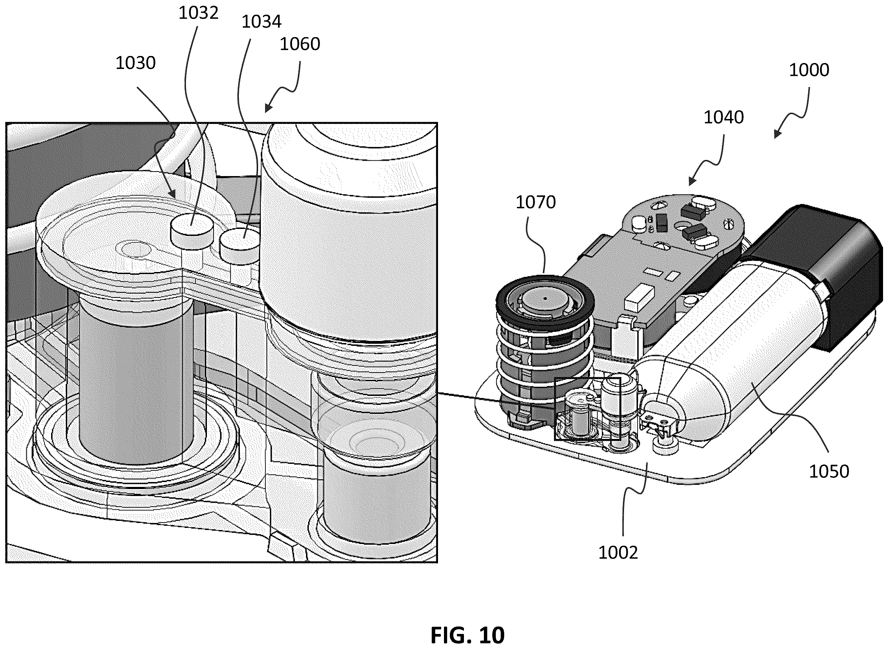

FIG. 10 is a schematic illustration of an example infusion device 1000 that is similar to infusion device 800 described above with respect to FIG. 8 except as described below, and can include many components with like numbering as infusion device 800. For example, the infusion device 1000 can include an adhesive 1002 for attaching the infusion device 1000 to a skin surface of a user. The infusion device 1000 can include a housing that houses various components including a reservoir 1050 (e.g., similar to reservoir 850), an insertion assembly 1070 (e.g., similar to insertion assembly 870), and an electronics assembly 1040 (e.g., similar to electronics assembly 840). However, infusion device 1000 can include an electrode arrangement 1030 that is located in or on a surface along a fluid flow path 1060 (e.g., channel, tubing, other conduit, etc.) between the reservoir 1050 and a cannula of the insertion assembly 1070, such that the working electrode 1032 and the counter electrode 1034 are in contact with the insulin formulation that travels from the reservoir 1050 to the cannula of the insertion assembly 1070. The electrode arrangement 1030 is in electrical communication with the electronics assembly 1040 such that the electrode arrangement 1030 (e.g., at least the working electrode 1032) can receive an input signal with multiple frequency components (e.g., as described above with respect to methods 200 and 500) that is generated by one or more processors (e.g., processor 110). Similar to that described above with respect to methods 200 and 500, the excited electrode arrangement 1030 can produce an output signal that can be analyzed to determine an impedance signature of the insulin formulation. For example, the output signal can be indicative of the insulin formulation (e.g., concentration of insulin, such as U100, U200, U500, etc.). In some variations, the concurrent multi-frequency impedance measurement with the electrode arrangement 1030 can be performed during a priming operation of the infusion device 1000. Furthermore, in some variations the infusion device 1000 can be configured to control delivery of the medicament based at least in part on the determination of the insulin formulation, similar to that described above with respect to method 500 (e.g., adjust dosage settings, providing an alert regarding the insulin concentration, enable or disable a lockout feature for delivery of the insulin formulation, etc.).

Although the example variations of patch infusion devices shown in FIGS. 8-10 include separate example locations of electrode arrangements, it should be understood that in some variations, a patch infusion device can include multiple sets of electrode arrangements in two or more locations. For example, a patch infusion device can include a first electrode arrangement at a reservoir outlet (e.g., similar to that shown in FIG. 8), a second electrode arrangement in the reservoir (e.g., similar to that shown in FIG. 9) and/or a third electrode arrangement in a fluid path between the reservoir and a cannula of the insertion assembly (e.g., similar to that shown in FIG. 10). For example, multiple sets of electrode arrangements can provide for extra redundancy in the system for performing impedance measurements (e.g., in case one electrode arrangement fails or provides inconclusive information) and/or can provide for increase accuracy in impedance measurement (e.g., by averaging the impedance measurements from multiple electrode arrangements). Additionally or alternatively, a patch infusion device can include an electrode arrangement in any suitable location that enables contact between the electrodes and the insulin formulation (e.g., anywhere along the fluid flow path between the reservoir and the insertion end of the cannula).

B. Tethered infusion systems

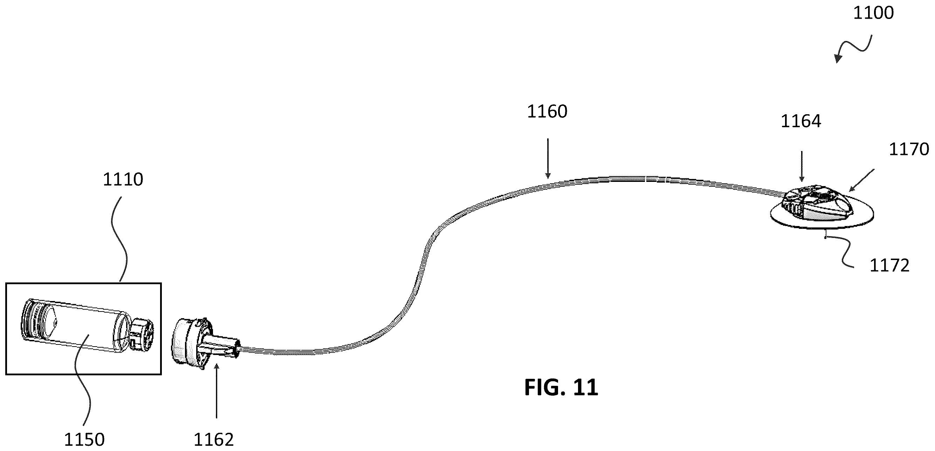

In some variations, an insulin delivery system includes a tethered infusion system including an infusion hub that is attachable to a skin surface of a user. For example, as shown in FIG. 11, a tethered infusion system 1100 includes an infusion pump 1110 having a reservoir 1150, an infusion hub 1170, and infusion tubing 1160 providing a fluidic conduit between the reservoir 1150 and the infusion hub 1170. The reservoir 1150 can be configured to hold an insulin formulation (or other suitable medicament) of any one of a range of insulin concentrations. In some variations, for example, the reservoir 1150 can be filled with an insulin formulation having a high concentration of insulin (e.g., U200, U500) that can be delivered in smaller increments across more doses, thereby enabling an extended wear lifetime of the patch infusion device. For example, in some variations the tethered infusion device can be configured for a wear lifetime of at least 5 days, at least 7 days, or at least 10 days. The reservoir 1150 can be prefilled and/or user-fillable by way of a fill port (not shown). The infusion hub 1170 can include an infusion cannula 1172 that is insertable into a user for conveying an insulin formulation into the user, and the infusion hub 1170 can include an adhesive surface to enable attachment of the infusion hub 1170 to a skin surface of the user. The infusion tubing 1160 can be connected at a first end to the reservoir 1150 via a tubing connector 1162, and connected at a second end to the infusion hub 1170 via a site connector 1164. The tethered infusion device 1100 can further include a pump (e.g., at the tubing connector 1162, etc.) to actuate the transfer of the insulin formulation from the reservoir to the infusion hub 1170. In operation, an insulin formulation in the reservoir 1150 can be transferred to the infusion hub 1170 through the infusion tubing 1160, and delivered to the user via the infusion cannula 1172. In some variations, the tethered infusion system 1100 can include an electrode arrangement configured to sense impedance measurements (e.g., in accordance with the methods 200 and 500 described herein).

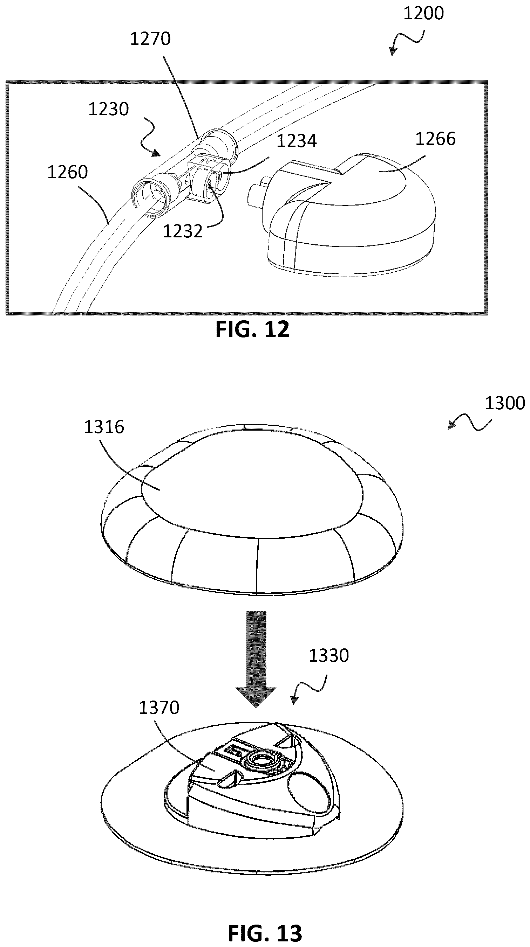

For example, FIG. 12 is a partial schematic illustration of a tethered infusion system 1200 similar to tethered infusion system 1100 except as described below. The tethered infusion system 1200 includes an electrode arrangement 1230 (e.g., similar to electrode arrangement 130) located in-line with the infusion tubing 1260, such that a working electrode 1232 and a counter electrode 1234 are in contact with an infusion formulation passing through the tubing. As shown in FIG. 12, the working electrode 1232 and the counter electrode 1234 can, for example, be included in a tubing sleeve that provides a tap into the flow path through the infusing tubing 1260.

A transmitter 1266 can be configured to couple to the tubing sleeve so as to receive and transmit impedance measurements and/or insulin concentration to a remote device. The transmitter 1266 can include a housing that functions to provide a fluidic seal around the tap and contain the infusion formulation. The electrode arrangement 1230 is in electrical communication with an electronics assembly (not shown), which can, for example, be within the transmitter 1266 (e.g., the transmitter 1266 can include an electronics assembly including a processor 110, a memory 120, and/or circuitry 140, etc.). For example, the electrode arrangement 1230 (e.g., at least the working electrode 1232) can receive an input signal with multiple frequency components (e.g., as described above with respect to methods 200 and 500) that is generated by one or more processors (e.g., processor 110). Similar to that described above with respect to methods 200 and 500, the excited electrode arrangement 1230 can produce an output signal that can be analyzed to determine an impedance signature of the insulin formulation. For example, the output signal can be indicative of the insulin formulation (e.g., concentration of insulin, such as U100, U200, U500, etc.). In some variations, the concurrent multi-frequency impedance measurement with the electrode arrangement 1230 can be performed during a priming operation of the infusion device 1200. In some variations, the transmitter 1266 can be configured to communicate the determined insulin formulation information (e.g., insulin concentration) to another portion of the tethered infusion device 1200 (e.g., pump). Furthermore, in some variations the infusion device 1200 can be configured to control delivery of the medicament based at least in part on the determination of the insulin formulation, similar to that described above with respect to method 500 (e.g., adjust dosage settings, providing an alert regarding the insulin concentration, enable or disable a lockout feature for delivery of the insulin formulation, etc.).

In some variations, it can be advantageous to locate the electrode arrangement 1230 in or around the infusion tubing 1260, as the infusion tubing 1260 can easily be made disposable so that updates to the electrode arrangement 1230 can be more easily implemented. For example, in some variations the electrode arrangement 1230 can be included in a clamshell-type or other connector 1270 configured to engage (e.g., clamp around) the infusion tubing 1260. Such a connector can include an interface in which the electrodes of the electrode arrangement 1230 can access contents within the infusion tubing 1260. For example, in some variations, the connector 1270 can include one or more needles or other suitable puncturing elements (e.g., the electrodes themselves), which can be configured to puncture the infusion tubing to enable contact between the electrodes and fluid within the infusion tubing 1260. In some variations, the connector can be disposable (e.g., disposed with the infusion tubing), while in some variations the connector can be removed from a first infusion tubing and reused in combination with a second infusion tubing (e.g., multi-use connector). In some variations, a housing of the connector 1270 can be reused across multiple infusion tubings, but the electrode arrangement 1230 can be removable from the connector 1270 and replaced between different infusion tubings.

As another example, FIG. 13 is a partial schematic illustration of a tethered infusion system 1300 similar to the tethered infusion system 1100 at the infusion hub end except as described below. The tethered infusion device 1300 includes an electrode arrangement 1330 (similar to electrode arrangement 130) located in or on the infusion hub 1370, with a working electrode and a counter electrode configured to be in contact with an insulin formulation that passes through the infusion hub 1370. The electrode arrangement 1330 is in electrical communication with an electronics assembly (not shown). For example, the electrode arrangement 1330 (e.g., at least the working electrode) can receive an input signal with multiple frequency components (e.g., as described above with respect to methods 200 and 500) that is generated by one or more processors (e.g., processor 110). Similar to that described above with respect to methods 200 and 500, the excited electrode arrangement 1330 can produce an output signal that can be analyzed to determine an impedance signature of the insulin formulation. For example, the output signal can be indicative of the insulin formulation (e.g., concentration of insulin, such as U100, U200, U500, etc.).

In some variations, the tethered infusion device 1300 can further include a wireless transmitter 1316 configured to communicate the determined insulin formulation information (e.g., insulin concentration) to another portion of the tethered infusion device 1300 (e.g., pump). In some variations, the transmitter 1316 can include the electronics assembly described above (e.g., the transmitter 1316 can include an electronics assembly including a processor 110, a memory 120, and/or circuitry 140, etc.). In some variations, the wireless transmitter 1316 can be detachably coupled to the infusion hub 1370. For example, the wireless transmitter 1316 can be coupled to the infusion hub 1370, and then decoupled from the infusion hub 1370 after the insulin concentration has been measured and communicated to another portion of the infusion device 1300 (e.g., pump). Furthermore, in some variations the infusion device 1300 can be configured to control delivery of the medicament based at least in part on the determination of the insulin formulation, similar to that described above with respect to method 500 (e.g., adjust dosage settings, providing an alert regarding the insulin concentration, enable or disable a lockout feature for delivery of the insulin formulation, etc.).

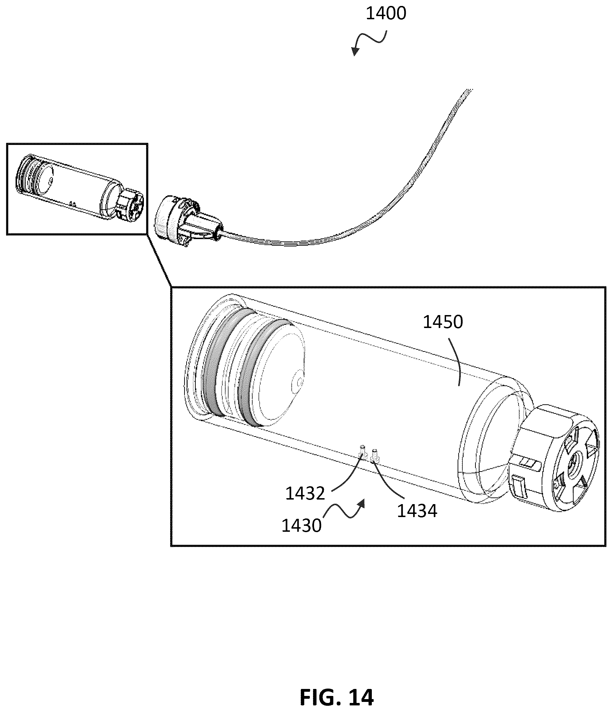

As another example, FIG. 14 is a partial schematic illustration of a tethered infusion system 1400 similar to the tethered infusion system 1110 except as described below. The tethered infusion system 1400 includes an electrode arrangement 1430 (similar to electrode arrangement 130) located in or on the reservoir 1450 (e.g., similar to reservoir 1150), with a working electrode 1432 and a counter electrode 1434 configured to be in contact with an insulin formulation held in the reservoir 1450. The electrode arrangement 1430 is in electrical communication with an electronics assembly (not shown) such that the electrode arrangement 1430 (e.g., at least the working electrode) can receive an input signal with multiple frequency components (e.g., as described above with respect to methods 200 and 500) that is generated by one or more processors (e.g., processor 110). Similar to that described above with respect to methods 200 and 500, the excited electrode arrangement 1430 can produce an output signal that can be analyzed to determine an impedance signature of the insulin formulation. For example, the output signal can be indicative of the insulin formulation (e.g., concentration of insulin, such as U100, U200, U500, etc.). In some variations, the concurrent multi-frequency impedance measurement with the electrode arrangement 1430 can be performed before or during a priming operation of the infusion device 1400. For example, in an example setup procedure, the pump of the infusion system 1400 can query an electrode arrangement 1430 located in or on the reservoir 1450, and once the insulin formulation (e.g., concentration) has been identified, the infusion system 1400 may be permitted to proceed through remaining setup processes (e.g., priming, infusion hub insertion, etc.).

In some variations, the output signal from the electrode arrangement 1430 can be communicated to an electronics assembly located adjacent to the reservoir (e.g., near a pump). However, in some variations, the tethered infusion system 1400 can further include a wireless transmitter (not shown) configured to communicate the determined insulin formulation information (e.g., insulin concentration) to another portion of the tethered infusion system 1400 (e.g., pump). Furthermore, in some variations the infusion system 1400 can be configured to control delivery of the medicament based at least in part on the determination of the insulin formulation, similar to that described above with respect to method 500 (e.g., adjust dosage settings, providing an alert regarding the insulin concentration, enable or disable a lockout feature for delivery of the insulin formulation, etc.).

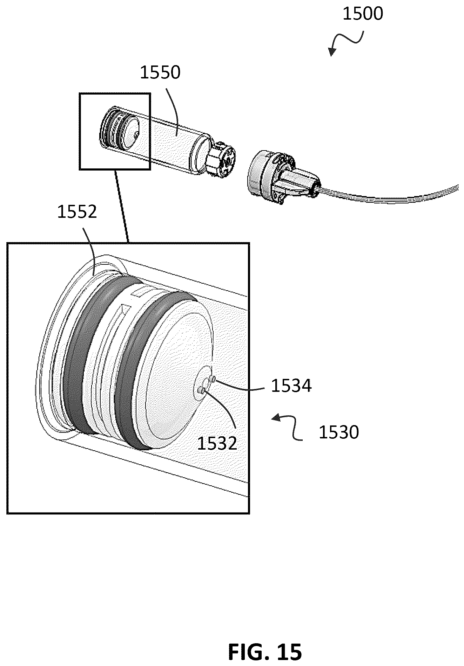

As another example, FIG. 15 is a partial schematic illustration of a tethered infusion system 1500 similar to the tethered infusion system 1110 except as described below. The tethered infusion system 1500 includes an electrode arrangement 1530 (similar to electrode arrangement 130) located in or on a stopper 1552 of the reservoir 1550, with a working electrode 1532 and a counter electrode 1534 configured to be in contact with an insulin formulation held in the reservoir 1550. The electrode arrangement 1530 is in electrical communication with an electronics assembly (not shown) such that the electrode arrangement 1530 (e.g., at least the working electrode) can receive an input signal with multiple frequency components (e.g., as described above with respect to methods 200 and 500) that is generated by one or more processors (e.g., processor 110). Similar to that described above with respect to methods 200 and 500, the excited electrode arrangement 1530 can produce an output signal that can be analyzed to determine an impedance signature of the insulin formulation. For example, the output signal can be indicative of the insulin formulation (e.g., concentration of insulin, such as U100, U200, U500, etc.). In some variations, the concurrent multi-frequency impedance measurement with the electrode arrangement 1530 can be performed before or during a priming operation of the infusion system 1500. For example, in an example setup procedure, the pump of the infusion system 1500 can query an electrode arrangement 1530 in or on the stopper 1552, and once the insulin formulation (e.g., concentration) has been identified, the infusion device 1500 may be permitted to proceed through remaining setup processes (e.g., priming, infusion hub insertion, etc.).

In some variations, the output signal from the electrode arrangement 1530 can be communicated to an electronics assembly located adjacent to the reservoir (e.g., near a pump). However, in some variations, the tethered infusion system 1500 can further include a wireless transmitter (not shown) configured to communicate the determined insulin formulation information (e.g., insulin concentration) to another portion of the tethered infusion system 1500 (e.g., pump). Furthermore, in some variations the infusion system 1500 can be configured to control delivery of the medicament based at least in part on the determination of the insulin formulation, similar to that described above with respect to method 500 (e.g., adjust dosage settings, providing an alert regarding the insulin concentration, enable or disable a lockout feature for delivery of the insulin formulation, etc.).

Although the example variations of tethered infusion systems shown in FIGS. 11-15 include separate example locations of electrode arrangements, it should be understood that in some variations, a tethered infusion system can include multiple sets of electrode arrangements in two or more locations. For example, a tethered infusion system can include a first electrode arrangement located in-line with infusion tubing (e.g., similar to that shown in FIG. 12), a second electrode arrangement located at an infusion hub (e.g., similar to that shown in FIG. 13), a third electrode arrangement located in a reservoir (e.g., similar to that shown in FIG. 14), and/or a fourth electrode arrangement located in a reservoir stopper (e.g., similar to that shown in FIG. 15). For example, multiple sets of electrode arrangements can provide for extra redundancy in the system for performing impedance measurements (e.g., in case one electrode arrangement fails or provides inconclusive information) and/or can provide for increase accuracy in impedance measurement (e.g., by averaging the impedance measurements from multiple electrode arrangements). Additionally or alternatively, a tethered infusion system can include an electrode arrangement in any suitable location that enables contact between the electrodes and the insulin formulation (e.g., anywhere along the fluid flow path between the reservoir and the insertion end of the cannula).

C. <u>Insulin pen</u>

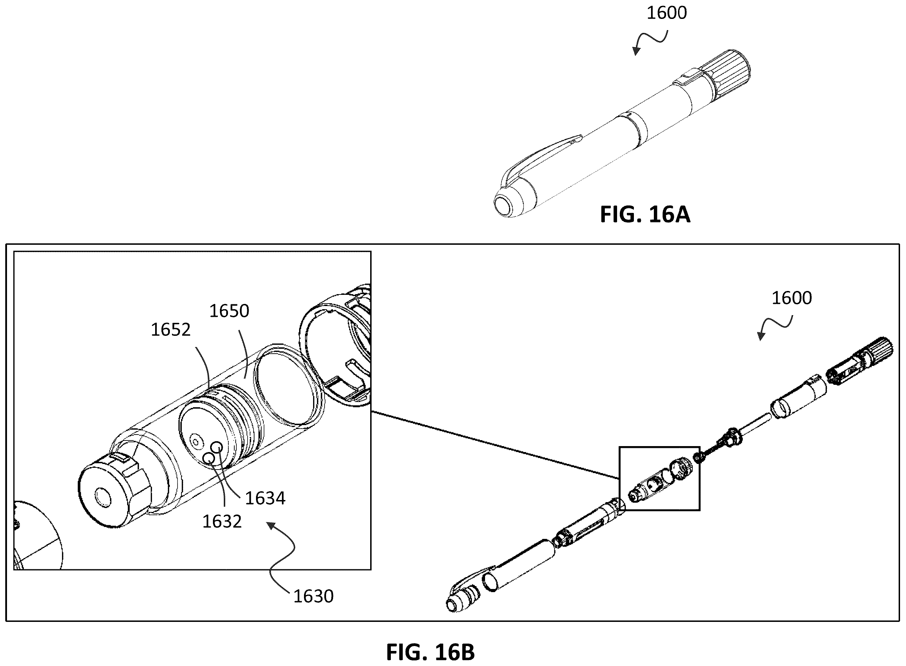

In some variations, an insulin delivery system includes a handheld infusion pen that includes a cannula insertable into a patient. For example, FIGS. 16A and 16B illustrate an example infusion pen device 1600 including a reservoir 1650 configured to hold an insulin formulation. The reservoir 1650 can further include a stopper 1652 configured to seal a volume of the insulin formulation within the reservoir 1650. The infusion pen device 1600 can further include an electrode arrangement 1630 having a working electrode 1632 and a counter electrode 1634 configured to be in contact with an insulin formulation in the reservoir 1650. For example, as shown in FIG. 16B, the working electrode 1632 and the counter electrode 1634 can be located on the stopper 1652 (similar as described above with respect to FIG. 15). As another example, the infusion pen device can additionally or alternatively include a working electrode 1632 and the counter electrode 1634 located in or on a wall of the reservoir 1650 (e.g., similar to that described above with respect to FIG. 14) and in configured to be in contact with an insulin formulation in the reservoir 1650. The electrode arrangement 1630 is in electrical communication with an electronics assembly (not shown) such that the electrode arrangement 1630 (e.g., at least the working electrode) can receive an input signal with multiple frequency components (e.g., as described above with respect to methods 200 and 500) that is generated by one or more processors (e.g., processor 110). Similar to that described above with respect to methods 200 and 500, the excited electrode arrangement 1630 can produce an output signal that can be analyzed to determine an impedance signature of the insulin formulation. For example, the output signal can be indicative of the insulin formulation (e.g., concentration of insulin, such as U100, U200, U500, etc.). In some variations, the concurrent multi-frequency impedance measurement with the electrode arrangement 1630 can be performed during a priming operation of the infusion device 1500. In some variations, the output signal from the electrode arrangement 1630 can be communicated to an electronics assembly located adjacent to the reservoir (e.g., near a pump).

However, in some variations, the tethered infusion device 1600 can further include a wireless transmitter (not shown) configured to communicate the determined insulin formulation information (e.g., insulin concentration) to another portion of the tethered infusion device 1500 (e.g., pump). Furthermore, in some variations the infusion device 1600 can be configured to control delivery of the medicament based at least in part on the determination of the insulin formulation, similar to that described above with respect to method 500 (e.g., adjust dosage settings, providing an alert regarding the insulin concentration, enable or disable a lockout feature for delivery of the insulin formulation, etc.).

D. Transfer guard

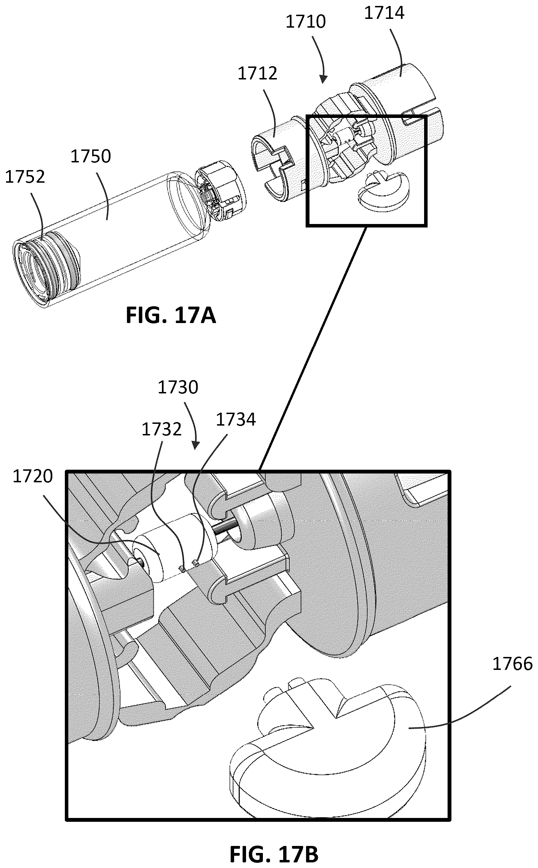

In some variations, a transfer guard can be used to aid transfer of an insulin formulation from an insulin vial to a reservoir (e.g., reservoir for a tethered infusion device). For example, FIGS. 17A and 17B illustrate an example transfer guard 1710 having a first end 1712 configured to couple to a reservoir 1750, and a second end 1714 configured to couple to an insulin vial (not shown). The reservoir 1750 can include a stopper 1752 that is configured to couple to a plunger rod. In use, one end of the reservoir 1750 can be coupled to the first end 1712 of the transfer guard 1710, and a plunger rod (e.g., similar to a syringe) can be coupled to an opposite end of the reservoir 1750 such that retraction of the plunger rod causes the reservoir 1750 to fill with air. Thereafter, the second end 1714 of the transfer guard 1710 can be coupled to an insulin vial including an insulin formulation, and the plunger rod can be depressed such that all of the air from the reservoir is injected into the insulin vial, thereby pressurizing the insulin vial. The assembled combination of the reservoir 1750, the transfer guard 1710, and the insulin vial can be inverted, and the plunger rod can then be against retracted to draw and fill the reservoir 1750 with insulin formulation from the insulin via a flow path passing between the second end 1714 and the first end 1712 of the transfer guard 1710.

In some variations, a transfer guard can include an electrode arrangement 1730, with a working electrode 1732 and a counter electrode 1734 configured to be in contact with the insulin formulation as it passes through the flow path between the second end 1714 and the first end 1712 of the transfer guard. For example, as shown in FIG. 17B, the electrode arrangement 1730 can be located in a module 1720 that is embedded or otherwise in-line or in contact with the flow path. The electrode arrangement 1730 is in electrical communication with an electronics assembly (not shown) such that the electrode arrangement 1730 (e.g., at least the working electrode) can receive an input signal with multiple frequency components (e.g., as described above with respect to methods 200 and 500) that is generated by one or more processors (e.g., processor 110). Similar to that described above with respect to methods 200 and 500, the excited electrode arrangement 1730 can produce an output signal that can be analyzed to determine an impedance signature of the insulin formulation. For example, the output signal can be indicative of the insulin formulation (e.g., concentration of insulin, such as U100, U200, U500, etc.).

In some variations, a wireless transmitter 1716 can be configured to couple to the module 1732 or otherwise be positioned proximate the electrode arrangement 1730. For example, the wireless transmitter 1766 can be connected to the electrode arrangement 1730 on the transfer guard 1710 while the transfer guard 1710 is still connected to the reservoir 1750. In some variations, the transmitter 1716 can include the electronics assembly described above (e.g., the transmitter 1716 can include an electronics assembly including a processor 110, a memory 120, and/or circuitry 140, etc.). After the insulin formulation type (e.g., concentration) is detected with the electrode arrangement, the transmitter 1766 can communicate the determined insulin formulation information (e.g., insulin concentration) to a portion of the tethered infusion system (e.g., pump), for example. Once the insulin formulation information is communicated, the transfer guard 1710 can be detached from the reservoir, and the transfer guard can be discarded.

Furthermore, in some variations an infusion device (e.g., tethered infusion device) associated with the transfer guard 1710 can be configured to control delivery of the medicament based at least in part on the determination of the insulin formulation, similar to that described above with respect to method 500 (e.g., adjust dosage settings, providing an alert regarding the insulin concentration, enable or disable a lockout feature for delivery of the insulin formulation, etc.).

EXAMPLES

The subject technology is illustrated, for example, according to various aspects described herein, including with reference to FIGS. 1A-16B. Various examples of the subject technology are described as numbered clauses (1, 2, 3, etc.) for convenience. These are provided as examples and do not limit the subject technology.

1. A method for identifying concentration of an analyte, the method comprising:

generating an input signal having a first set of frequency components across a plurality of frequencies;
exciting an electrode arrangement with the input signal, wherein the electrode arrangement is in contact with the analyte;
receiving an output signal from the excited electrode arrangement, the output signal having a second set of frequency components across the plurality of frequencies;
determining an impedance signature of the analyte across the plurality of frequencies, based on the first and second sets of frequency components; and
determining a concentration of the analyte based on the impedance signature.

2. The method of clause 1, wherein the input signal is a non-sinusoidal wave.

3. The method of clause 1 or 2, wherein the input signal is a square wave, a step function, or a triangle wave.

4. The method of any one of clauses 1-3, wherein the input signal has an operating excitation voltage range of +1.23 V to -1.23 V.

5. The method of any one of clauses 1-3, wherein the input signal has an operating excitation voltage range of +600 mV to -600 mV.

6. The method of any one of clauses 1-3, wherein the input signal has an operating excitation voltage range of +400 mV to -400 mV.

7. The method of any one of clauses 1-6, wherein determining the impedance signature of the analyte comprises determining an impedance spectrum of the analyte.

8. The method of any one of clauses 1-7, wherein determining the impedance signature of the analyte comprises:

decomposing the input signal into the first set of frequency components;
decomposing the output signal into the second set of frequency components; and
determining an impedance of the analyte at each of the plurality of frequencies based on a comparison of the first and second sets of frequency components.

9. The method of clause 8, wherein decomposing the input signal comprises applying a first Fourier transform to the input signal, and decomposing the output signal comprises applying a second Fourier transform to the output signal.

10. The method of any one of clauses 1-9, wherein determining the concentration of the analyte comprises comparing the impedance signature to a lookup table stored in one or more memory devices.

11. The method of any one of clauses 1-10, wherein determining the concentration of the analyte comprises determining compositional information of the analyte.

12. The method of any one of clauses 1-11, wherein the electrode arrangement is in contact with a fluid path of a fluid including the analyte.

13. The method of any one of clauses 1-12, wherein the electrode arrangement comprises two or more electrodes.

14. The method of any one of clauses 1-13, wherein one or more electrodes includes at least one of a catalyst or enzyme.

15. The method of any one of clauses 1-14, wherein the electrode arrangement is in an analyte delivery device.

16. The method of clause 15, further comprising controlling delivery of the analyte to a user with the analyte delivery device, based at least in part on the determined concentration of the analyte.

17. The method of clause 16, wherein controlling delivery of the analyte comprises:

determining a volume of fluid including the analyte to be delivered to the user; and
delivering the determined volume of fluid to the user.

18. The method of clause 16, wherein controlling delivery of the analyte comprises providing an alert communicating an indication of the determined concentration of the analyte.

19. The method of any one of clauses 1-18, wherein the electrode arrangement is in an analyte monitoring device.

20. The method of any one of clauses 1-19, wherein the analyte is insulin.

21. A system for measuring concentration of an analyte, the system comprising:

an electrode arrangement in contact with a fluid including the analyte;
a processor; and
a memory device operably coupled to the processor and storing instructions that, when executed by the processor, cause the system to perform operations comprising:

generating an input signal having a first set of frequency components across a plurality of frequencies;
exciting the electrode arrangement with the input signal;
receiving an output signal from the excited electrode arrangement, the output

signal having a second set of frequency components across the plurality of frequencies;

determining an impedance signature of the analyte across the plurality of frequencies, based on the first and second sets of frequency components; and
determining a concentration of the analyte based on the impedance signature.

22. The system of clause 21, wherein the input signal is a non-sinusoidal wave.

23. The system of clause 21 or 22, wherein the input signal is a square wave or a triangle wave.

24. The system of any one of clauses 21-23, wherein the input signal has an operating excitation voltage range of +1.23 V to -1.23 V.

25. The system of any one of clauses 21-23, wherein the input signal has an operating excitation voltage range of +600 mV to -600 mV.

26. The system of any one of clauses 21-23, wherein the input signal has an operating excitation voltage range of +400 mV to -400 mV.

27. The system of any one of clauses 21-26, wherein determining the impedance signature of the analyte comprises determining an impedance spectrum of the analyte.

28. The system of any one of clauses 21-27, determining the impedance signature of the analyte comprises:

decomposing the input signal into the first set of frequency components;
decomposing the output signal into the second set of frequency components; and
determining an impedance of the analyte at each of the plurality of frequencies based on a comparison of the first and second sets of frequency components.

29. The system of clause 28, wherein decomposing the input signal comprises applying a first Fourier transform to the input signal, and decomposing the output signal comprises applying a second Fourier transform to the output signal.

30. The system of any one of clauses 21-29, wherein determining the concentration of the analyte comprises comparing the impedance signature to a lookup table stored in one or more memory devices.

31. The system of any one of clauses 21-30, wherein determining the concentration of the analyte comprises determining compositional information of the analyte.

32. The system of any one of clauses 21-31, wherein the electrode arrangement comprises a plurality of electrodes comprising a working electrode and a counter electrode.

33. The system of clause 32, wherein the plurality of electrodes further comprises a reference electrode.

34. The system of clause 32 or 33, wherein at least one electrode of the plurality of electrodes comprises at least one of a catalyst or an enzyme.

35. The system of any one of clauses 21-34, wherein the system comprises an analyte delivery device.

36. The system of clause 35, wherein the analyte delivery device comprises an insulin pump.

37. The system of clause 36, wherein the analyte delivery device comprises a patch pump device.

38. The system of clause 37, wherein the analyte delivery device comprises a tethered pump device.

39. The system of clause 38, wherein the analyte delivery device comprises an insulin pen.

40. The system of any one of clauses 21-39, wherein the system comprises an analyte monitoring device.

41. The system of clause 40, wherein the analyte monitoring device comprises a continuous glucose monitoring device.

42. A method for characterizing a substance, the method comprising:

generating an input signal having a first set of frequency components across a plurality of frequencies;
exciting an electrode arrangement with the input signal, wherein the electrode arrangement is in contact with the substance;
receiving an output signal from the excited electrode arrangement, the output signal having a second set of frequency components across the plurality of frequencies;
determining an impedance signature of the substance across the plurality of frequencies, based on the first and second sets of frequency components; and
characterizing the substance based on the impedance signature.

43. The method of clause 42, wherein the input signal is a non-sinusoidal wave.

44. The method of clause 42 or 43, wherein the input signal is a square wave or a triangle wave.

45. The method of any one of clauses 42-44, wherein the input signal has an operating excitation voltage range of +1.23 V to -1.23 V.

46. The method of any one of clauses 42-44, wherein the input signal has an operating excitation voltage range of +600 mV to -600 mV.

47. The method of any one of clauses 42-44, wherein the input signal has an operating excitation voltage range of +400 mV to -400 mV.

48. The method of any one of clauses 42-47, wherein determining the impedance signature of the analyte comprises determining an impedance spectrum of the analyte.

49. The method of any one of clauses 42-48, wherein determining the impedance signature of the analyte comprises:

decomposing the input signal into the first set of frequency components;
decomposing the output signal into the second set of frequency components; and
determining an impedance of the analyte at each of the plurality of frequencies based on a comparison of the first and second sets of frequency components.

50. The method of clause 49, wherein decomposing the input signal comprises applying a first Fourier transform to the input signal, and decomposing the output signal comprises applying a second Fourier transform to the output signal.

51. The method of any one of clauses 42-50, wherein characterizing the substance comprises comparing the impedance signature to a lookup table stored in one or more memory devices.

52. The method of any one of clauses 42-51, wherein the substance comprises a medicament to be delivered to a patient, and wherein the method further comprises controlling delivery of the medicament to the patient based on the characterization of the substance.

53. A system for characterizing a substance, the system comprising:

an electrode arrangement in contact with the substance;
a processor; and
a memory device operably coupled to the processor and storing instructions that, when executed by the processor, cause the system to perform operations comprising:

generating an input signal having a first set of frequency components across a plurality of frequencies;
exciting the electrode arrangement with the input signal, wherein the electrode arrangement is in contact with the substance;
receiving an output signal from the excited electrode arrangement, the output signal having a second set of frequency components across the plurality of frequencies;
determining an impedance signature of the substance across the plurality of frequencies, based on the first and second sets of frequency components; and
characterizing the substance based on the impedance signature.

54. The system of clause 53, wherein the input signal is a non-sinusoidal wave.

55. The system of clause 53 or 54, wherein the input signal is a square wave or a triangle wave.

56. The system of any one of clauses 53-55, wherein the input signal has an operating excitation voltage range of +1.23 V to -1.23 V.

57. The system of any one of clauses 53-55, wherein the input signal has an operating excitation voltage range of +600 mV to -600 mV.

58. The method of any one of clauses 53-55, wherein the input signal has an operating excitation voltage range of +400 mV to -400 mV.

59. The system of any one of clauses 53-58, wherein determining the impedance signature of the analyte comprises determining an impedance spectrum of the analyte.

60. The system of any one of clauses 53-59, wherein determining the impedance signature of the analyte comprises:

decomposing the input signal into the first set of frequency components;
decomposing the output signal into the second set of frequency components; and
determining an impedance of the analyte at each of the plurality of frequencies based on a comparison of the first and second sets of frequency components.

61. The system of clause 60, wherein decomposing the input signal comprises applying a first Fourier transform to the input signal, and decomposing the output signal comprises applying a second Fourier transform to the output signal.

62. The system of any one of clauses 53-61, wherein characterizing the substance comprises comparing the impedance signature to a lookup table stored in one or more memory devices.

63. The system of any one of clauses 53-62, wherein the substance comprises a medicament to be delivered to a patient, and wherein the instructions, when executed by the processor, cause the system to perform operations comprising controlling delivery of the medicament to the patient based on the characterization of the substance.

64. A method for configuring an insulin delivery system, the method comprising:

generating an input signal having a first set of frequency components across a plurality of frequencies;
exciting an electrode arrangement with the input signal, wherein the electrode arrangement is in contact with an insulin formulation in an infusion device;

receiving an output signal from the excited electrode arrangement, the output signal having a second set of frequency components across the plurality of frequencies;

determining an impedance signature of the insulin formulation across the plurality of frequencies, based on the first and second sets of frequency components;

determining an insulin concentration of the insulin formulation based on the impedance signature; and

configuring the infusion device based on the determined insulin concentration.

65. The method of clause 64, wherein configuring the infusion device comprises preventing the infusion device from delivering the insulin formulation.

66. The method of clause 64 or 65, wherein configuring the infusion device comprises setting delivery parameters based on the determined insulin concentration.

67. The method of any one of clauses 64-66, wherein configuring the infusion device comprises providing a prompt to a user to update delivery parameters.

68. The method of any one of clauses 64-67, wherein the infusion device comprises a patch pump comprising a reservoir configured to hold the insulin formulation.

69. The method of clause 68, wherein at least a portion of the electrode arrangement is located at an outlet of the reservoir.

70. The method of clause 68 or 69, wherein at least a portion of the electrode arrangement is located in a body of the reservoir.

71. The method of any one of clauses 68-70, wherein the patch pump comprises a cannula configured to infuse the insulin formulation into a user, and wherein at least a portion of the electrode arrangement is located in a fluid flow path between the reservoir and the cannula.

72. The method of any one of clauses 64-67, wherein the infusion device comprises a tethered infusion system comprising a reservoir configured to hold the insulin formulation.

73. The method of clause 72, wherein at least a portion of the electrode arrangement is located at an outlet of the reservoir.

74. The method of clause 72 or 73, wherein the tethered infusion system comprises an infusion tubing fluidically coupled to the reservoir, wherein at least a portion of the electrode arrangement is located inline with the infusion tubing.

75. The method of any one of clauses 72-74, wherein the tethered infusion system comprises an infusion hub fluidically coupled to the reservoir, wherein at least a portion of the electrode arrangement is located in the infusion hub.

76. The method of any one of clauses 72-75, wherein at least a portion of the electrode arrangement is located in a body of the reservoir.

77. The method of any one of clauses 72-76, wherein at least a portion of the electrode arrangement is located in a stopper of the reservoir.

78. The method of any one of clauses 64-77, wherein the infusion device comprises an infusion pen comprising a reservoir configured to hold the insulin formulation.

79. The method of clause 78, wherein at least a portion of the electrode arrangement is located in a body of the reservoir.

80. The method of clause 78 or 79, wherein at least a portion of the electrode arrangement is located in a stopper of the reservoir.

81. An insulin delivery system comprising:

an infusion device comprising a reservoir configured to receive an insulin formulation;
an electrode arrangement in contact with the insulin formulation;
a processor; and
a memory device operably coupled to the processor and storing instructions that, when executed by the processor, cause the insulin delivery system to perform operations comprising:

generating an input signal having a first set of frequency components across a plurality of frequencies;
exciting the electrode arrangement with the input signal, wherein the electrode arrangement is in contact with the insulin formulation;
receiving an output signal from the excited electrode arrangement, the output signal having a second set of frequency components across the plurality of frequencies;
determining an impedance signature of the insulin formulation across the plurality of frequencies, based on the first and second sets of frequency components;
characterizing the insulin formulation based on the impedance signature; and
configuring the infusion device based on the characterization of the insulin formulation.

82. The system of clause 81, wherein configuring the infusion device comprises preventing the infusion device from delivering the insulin formulation.

83. The system of clause 81, wherein configuring the infusion device comprises setting delivery parameters based on the determined insulin concentration.

84. The system of any one of clauses 81-83, wherein configuring the infusion device comprises providing a prompt to a user to update delivery parameters.

85. The system of any one of clauses 81-84, wherein the infusion device comprises a patch pump comprising a reservoir configured to hold the insulin formulation.

86. The system of clause 85, wherein at least a portion of the electrode arrangement is located at an outlet of the reservoir.

87. The system of clause 85 or 86, wherein at least a portion of the electrode arrangement is located in a body of the reservoir.

88. The system of any one of clauses 85-87, wherein the patch pump comprises a cannula configured to infuse the insulin formulation into a user, and wherein at least a portion of the electrode arrangement is located in a fluid flow path between the reservoir and the cannula.

89. The system of any one of clauses 81-84, wherein the infusion device comprises a tethered infusion system comprising a reservoir configured to hold the insulin formulation.

90. The system of clause 89, wherein at least a portion of the electrode arrangement is located at an outlet of the reservoir.

91. The system of clause 89 or 90, wherein the tethered infusion system comprises an infusion tubing fluidically coupled to the reservoir, wherein at least a portion of the electrode arrangement is located inline with the infusion tubing.

92. The system of any one of clauses 89-91, wherein the tethered infusion system comprises an infusion hub fluidically coupled to the reservoir, wherein at least a portion of the electrode arrangement is located in the infusion hub.

93. The system of any one of clauses 89-92, wherein at least a portion of the electrode arrangement is located in a body of the reservoir.

94. The system of any one of clauses 89-93, wherein at least a portion of the electrode arrangement is located in a stopper of the reservoir.

95. The system of any one of clauses 89-94, wherein the infusion device comprises an infusion pen comprising a reservoir configured to hold the insulin formulation.

96. The system of clause 95, wherein at least a portion of the electrode arrangement is located in a body of the reservoir.

97. The system of clause 95 or 96, wherein at least a portion of the electrode arrangement is located in a stopper of the reservoir.

Conclusion

Although many of the variations are described above with respect to systems, devices, and methods for detecting concentration of an analyte such as insulin, the technology is applicable to other applications and/or other approaches, such as detecting concentration of other analytes, monitoring the integrity of an electrode-tissue interface, detecting disease state of tissue, determining tissue composition, detecting failure of electrical components, and/or the like. Moreover, other variations in addition to those described herein are within the scope of the technology. Additionally, several other variations of the technology can have different configurations, components, or procedures than those described herein. A person of ordinary skill in the art, therefore, will accordingly understand that the technology can have other variations with additional elements, or the technology can have other variations without several of the features shown and described above with reference to FIGS. 1A-16B.

The descriptions of variations of the technology are not intended to be exhaustive or to limit the technology to the precise form disclosed above. Where the context permits, singular or plural terms may also include the plural or singular term, respectively. Although specific variations of, and examples for, the technology are described above for illustrative purposes, various equivalent modifications are possible within the scope of the technology, as those skilled in the relevant art will recognize. For example, while steps are presented in a given order, alternative variations may perform steps in a different order. The various variations described herein may also be combined to provide further variations.

As used herein, the terms "generally," "substantially," "about," and similar terms are used as terms of approximation and not as terms of degree, and are intended to account for the inherent variations in measured or calculated values that would be recognized by those of ordinary skill in the art.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific variations have been described herein for purposes of illustration, but that various modifications may be made without deviating from the technology. Further, while advantages associated with certain variations of the technology have been described in the context of those variations, other variations may also exhibit such advantages, and not all variations need necessarily exhibit such advantages to fall within the scope of the technology. Accordingly, the disclosure and associated technology can encompass other variations not expressly shown or described herein.

Further disclosed herein is the subject-matter of the following items:

1. A method for identifying concentration of an analyte, the method comprising:

generating an input signal having a first set of frequency components across a plurality of frequencies;
exciting an electrode arrangement with the input signal, wherein the electrode arrangement is in contact with the analyte;
receiving an output signal from the excited electrode arrangement, the output signal having a second set of frequency components across the plurality of frequencies;
determining an impedance signature of the analyte across the plurality of frequencies, based on the first and second sets of frequency components; and
determining a concentration of the analyte based on the impedance signature.

2. The method of item 1, wherein the input signal is a non-sinusoidal wave.

3. The method of item 1 or 2, wherein the input signal is a square wave, a step function, or a triangle wave.

4. The method of any one of items 1-3, wherein the input signal has an operating excitation voltage range of +1.23 V to -1.23 V.

5. The method of any one of items 1-3 or of any one of items 1-4, wherein the input signal has an operating excitation voltage range of +600 mV to -600 mV.

6. The method of any one of items 1-3 or of any one of items 1-5, wherein the input signal has an operating excitation voltage range of +400 mV to -400 mV.

7. The method of any one of items 1-6, wherein determining the impedance signature of the analyte comprises determining an impedance spectrum of the analyte.

8. The method of any one of items 1-7, wherein determining the impedance signature of the analyte comprises:

decomposing the input signal into the first set of frequency components;
decomposing the output signal into the second set of frequency components; and
determining an impedance of the analyte at each of the plurality of frequencies based on a comparison of the first and second sets of frequency components.

9. The method of item 8 or of any one of items 1-8, wherein decomposing the input signal comprises applying a first Fourier transform to the input signal, and decomposing the output signal comprises applying a second Fourier transform to the output signal.

10. The method of any one of items 1-9, wherein determining the concentration of the analyte comprises comparing the impedance signature to a lookup table stored in one or more memory devices.

11. The method of any one of items 1-10, wherein determining the concentration of the analyte comprises determining compositional information of the analyte.

12. The method of any one of items 1-11, wherein the electrode arrangement is in contact with a fluid path of a fluid including the analyte.

13. The method of any one of items 1-12, wherein the electrode arrangement comprises two or more electrodes.

14. The method of any one of items 1-13, wherein one or more electrodes includes at least one of a catalyst or enzyme.

15. The method of any one of items 1-14, wherein the electrode arrangement is in an analyte delivery device.

16. The method of item 15 or of any one of items 1-15, further comprising controlling delivery of the analyte to a user with the analyte delivery device, based at least in part on the determined concentration of the analyte.

17. The method of item 16 or of any one of items 1-16, wherein controlling delivery of the analyte comprises:

determining a volume of fluid including the analyte to be delivered to the user; and
delivering the determined volume of fluid to the user.

18. The method of item 16 or of any one of items 1-17, wherein controlling delivery of the analyte comprises providing an alert communicating an indication of the determined concentration of the analyte.

19. The method of any one of items 1-18, wherein the electrode arrangement is in an analyte monitoring device.

20. The method of any one of items 1-19, wherein the analyte is insulin.

21. A system for measuring concentration of an analyte, the system comprising:

an electrode arrangement in contact with a fluid including the analyte;
a processor; and
a memory device operably coupled to the processor and storing instructions that, when executed by the processor, cause the system to perform operations comprising:

generating an input signal having a first set of frequency components across a plurality of frequencies;

exciting the electrode arrangement with the input signal;
receiving an output signal from the excited electrode arrangement, the output

signal having a second set of frequency components across the plurality of frequencies;

determining an impedance signature of the analyte across the plurality of frequencies, based on the first and second sets of frequency components; and
determining a concentration of the analyte based on the impedance signature.

22. The system of item 21, wherein the input signal is a non-sinusoidal wave.

23. The system of item 21 or 22, wherein the input signal is a square wave or a triangle wave.

24. The system of any one of items 21-23, wherein the input signal has an operating excitation voltage range of +1.23 V to -1.23 V.

25. The system of any one of items 21-23 or of any one of items 21-24, wherein the input signal has an operating excitation voltage range of +600 mV to -600 mV.

26. The system of any one of items 21-23 or of any one of items 21-25, wherein the input signal has an operating excitation voltage range of +400 mV to -400 mV.

27. The system of any one of items 21-26, wherein determining the impedance signature of the analyte comprises determining an impedance spectrum of the analyte.

28. The system of any one of items 21-27, determining the impedance signature of the analyte comprises:

decomposing the input signal into the first set of frequency components;
decomposing the output signal into the second set of frequency components; and
determining an impedance of the analyte at each of the plurality of frequencies based on a comparison of the first and second sets of frequency components.

29. The system of item 28 or of any one of items 21-28, wherein decomposing the input signal comprises applying a first Fourier transform to the input signal, and decomposing the output signal comprises applying a second Fourier transform to the output signal.

30. The system of any one of items 21-29, wherein determining the concentration of the analyte comprises comparing the impedance signature to a lookup table stored in one or more memory devices.

31. The system of any one of items 21-30, wherein determining the concentration of the analyte comprises determining compositional information of the analyte.

32. The system of any one of items 21-31, wherein the electrode arrangement comprises a plurality of electrodes comprising a working electrode and a counter electrode.

33. The system of item 32 or of any one of items 21-32, wherein the plurality of electrodes further comprises a reference electrode.

34. The system of item 32 or 33 or of any one of items 21-33, wherein at least one electrode of the plurality of electrodes comprises at least one of a catalyst or an enzyme.

35. The system of any one of items 21-34, wherein the system comprises an analyte delivery device.

36. The system of item 35 or of any one of items 21-35, wherein the analyte delivery device comprises an insulin pump.

37. The system of item 36 or of any one of items 21-36, wherein the analyte delivery device comprises a patch pump device.

38. The system of item 37 or of any one of items 21-37, wherein the analyte delivery device comprises a tethered pump device.

39. The system of item 38 or of any one of items 21-38, wherein the analyte delivery device comprises an insulin pen.

40. The system of any one of items 21-39, wherein the system comprises an analyte monitoring device.

41. The system of item 40 or of any one of items 21-40, wherein the analyte monitoring device comprises a continuous glucose monitoring device.

42. A method for characterizing a substance, the method comprising:

generating an input signal having a first set of frequency components across a plurality of frequencies;
exciting an electrode arrangement with the input signal, wherein the electrode arrangement is in contact with the substance;
receiving an output signal from the excited electrode arrangement, the output signal having a second set of frequency components across the plurality of frequencies;
determining an impedance signature of the substance across the plurality of frequencies, based on the first and second sets of frequency components; and
characterizing the substance based on the impedance signature.

43. The method of item 42, wherein the input signal is a non-sinusoidal wave.

44. The method of item 42 or 43, wherein the input signal is a square wave or a triangle wave.

45. The method of any one of items 42-44, wherein the input signal has an operating excitation voltage range of +1.23 V to -1.23 V.

46. The method of any one of items 42-44 or of any one of items 42-45, wherein the input signal has an operating excitation voltage range of +600 mV to -600 mV.

47. The method of any one of items 42-44 or of any one of items 42-46, wherein the input signal has an operating excitation voltage range of +400 mV to -400 mV.

48. The method of any one of items 42-47, wherein determining the impedance signature of the analyte comprises determining an impedance spectrum of the analyte.

49. The method of any one of items 42-48, wherein determining the impedance signature of the analyte comprises:

decomposing the input signal into the first set of frequency components;
decomposing the output signal into the second set of frequency components; and
determining an impedance of the analyte at each of the plurality of frequencies based on a comparison of the first and second sets of frequency components.

50. The method of item 49 or of any one of items 42-49, wherein decomposing the input signal comprises applying a first Fourier transform to the input signal, and decomposing the output signal comprises applying a second Fourier transform to the output signal.

51. The method of any one of items 42-50, wherein characterizing the substance comprises comparing the impedance signature to a lookup table stored in one or more memory devices.

52. The method of any one of items 42-51, wherein the substance comprises a medicament to be delivered to a patient, and wherein the method further comprises controlling delivery of the medicament to the patient based on the characterization of the substance.

53. A system for characterizing a substance, the system comprising:

an electrode arrangement in contact with the substance;

a processor; and

a memory device operably coupled to the processor and storing instructions that, when executed by the processor, cause the system to perform operations comprising:

generating an input signal having a first set of frequency components across a plurality of frequencies;
exciting the electrode arrangement with the input signal, wherein the electrode arrangement is in contact with the substance;
receiving an output signal from the excited electrode arrangement, the output signal having a second set of frequency components across the plurality of frequencies;
determining an impedance signature of the substance across the plurality of frequencies, based on the first and second sets of frequency components; and
characterizing the substance based on the impedance signature.

54. The system of item 53, wherein the input signal is a non-sinusoidal wave.

55. The system of item 53 or 54, wherein the input signal is a square wave or a triangle wave.

56. The system of any one of items 53-55, wherein the input signal has an operating excitation voltage range of +1.23 V to -1.23 V.

57. The system of any one of items 53-55 or of any one of items 53-56, wherein the input signal has an operating excitation voltage range of +600 mV to -600 mV.

58. The method of any one of items 53-55 or of any one of items 53-57, wherein the input signal has an operating excitation voltage range of +400 mV to -400 mV.

59. The system of any one of items 53-58, wherein determining the impedance signature of the analyte comprises determining an impedance spectrum of the analyte.

60. The system of any one of items 53-59, wherein determining the impedance signature of the analyte comprises:

decomposing the input signal into the first set of frequency components;
decomposing the output signal into the second set of frequency components; and
determining an impedance of the analyte at each of the plurality of frequencies based on a comparison of the first and second sets of frequency components.

61. The system of item 60 or of any one of items 53-60, wherein decomposing the input signal comprises applying a first Fourier transform to the input signal, and decomposing the output signal comprises applying a second Fourier transform to the output signal.

62. The system of any one of items 53-61, wherein characterizing the substance comprises comparing the impedance signature to a lookup table stored in one or more memory devices.

63. The system of any one of items 53-62, wherein the substance comprises a medicament to be delivered to a patient, and wherein the instructions, when executed by the processor, cause the system to perform operations comprising controlling delivery of the medicament to the patient based on the characterization of the substance.

64. A method for configuring an insulin delivery system, the method comprising:

generating an input signal having a first set of frequency components across a plurality of frequencies;
exciting an electrode arrangement with the input signal, wherein the electrode arrangement is in contact with an insulin formulation in an infusion device;
receiving an output signal from the excited electrode arrangement, the output signal having a second set of frequency components across the plurality of frequencies;
determining an impedance signature of the insulin formulation across the plurality of frequencies, based on the first and second sets of frequency components;
determining an insulin concentration of the insulin formulation based on the impedance signature; and
configuring the infusion device based on the determined insulin concentration.

65. The method of item 64, wherein configuring the infusion device comprises preventing the infusion device from delivering the insulin formulation.

66. The method of item 64 or 65, wherein configuring the infusion device comprises setting delivery parameters based on the determined insulin concentration.

67. The method of any one of items 64-66, wherein configuring the infusion device comprises providing a prompt to a user to update delivery parameters.

68. The method of any one of items 64-67, wherein the infusion device comprises a patch pump comprising a reservoir configured to hold the insulin formulation.

69. The method of item 68 or of any one of items 64-68, wherein at least a portion of the electrode arrangement is located at an outlet of the reservoir.

70. The method of item 68 or 69 or of any one of items 64-69, wherein at least a portion of the electrode arrangement is located in a body of the reservoir.

71. The method of any one of items 68-70 or of any one of items 64-70, wherein the patch pump comprises a cannula configured to infuse the insulin formulation into a user, and wherein at least a portion of the electrode arrangement is located in a fluid flow path between the reservoir and the cannula.

72. The method of any one of items 64-67 or of any one of items 64-71, wherein the infusion device comprises a tethered infusion system comprising a reservoir configured to hold the insulin formulation.

73. The method of item 72 or of any one of items 64-72, wherein at least a portion of the electrode arrangement is located at an outlet of the reservoir.

74. The method of item 72 or 73 or of any one of items 64-73, wherein the tethered infusion system comprises an infusion tubing fluidically coupled to the reservoir, wherein at least a portion of the electrode arrangement is located inline with the infusion tubing.

75. The method of any one of items 72-74 or of any one of items 64-74, wherein the tethered infusion system comprises an infusion hub fluidically coupled to the reservoir, wherein at least a portion of the electrode arrangement is located in the infusion hub.

76. The method of any one of items 72-75 or of any one of items 64-75, wherein at least a portion of the electrode arrangement is located in a body of the reservoir.

77. The method of any one of items 72-76 or of any one of items 64-76, wherein at least a portion of the electrode arrangement is located in a stopper of the reservoir.

78. The method of any one of items 64-77, wherein the infusion device comprises an infusion pen comprising a reservoir configured to hold the insulin formulation.

79. The method of item 78 or of any one of items 64-78, wherein at least a portion of the electrode arrangement is located in a body of the reservoir.

80. The method of item 78 or 79 or of any one of items 64-79, wherein at least a portion of the electrode arrangement is located in a stopper of the reservoir.

81. An insulin delivery system comprising:

an infusion device comprising a reservoir configured to receive an insulin formulation;
an electrode arrangement in contact with the insulin formulation;
a processor; and
a memory device operably coupled to the processor and storing instructions that, when executed by the processor, cause the insulin delivery system to perform operations comprising:

generating an input signal having a first set of frequency components across a plurality of frequencies;

exciting the electrode arrangement with the input signal, wherein the electrode arrangement is in contact with the insulin formulation;

receiving an output signal from the excited electrode arrangement, the output signal having a second set of frequency components across the plurality of frequencies;

determining an impedance signature of the insulin formulation across the plurality of frequencies, based on the first and second sets of frequency components;

characterizing the insulin formulation based on the impedance signature; and

configuring the infusion device based on the characterization of the insulin formulation.

82. The system of item 81, wherein configuring the infusion device comprises preventing the infusion device from delivering the insulin formulation.

83. The system of item 81 or 82, wherein configuring the infusion device comprises setting delivery parameters based on the determined insulin concentration.

84. The system of any one of items 81-83, wherein configuring the infusion device comprises providing a prompt to a user to update delivery parameters.

85. The system of any one of items 81-84, wherein the infusion device comprises a patch pump comprising a reservoir configured to hold the insulin formulation.

86. The system of item 85 or of any one of items 81-85, wherein at least a portion of the electrode arrangement is located at an outlet of the reservoir.

87. The system of item 85 or 86 or of any one of items 81-86, wherein at least a portion of the electrode arrangement is located in a body of the reservoir.

88. The system of any one of items 85-87 or of any one of items 81-87, wherein the patch pump comprises a cannula configured to infuse the insulin formulation into a user, and wherein at least a portion of the electrode arrangement is located in a fluid flow path between the reservoir and the cannula.

89. The system of any one of items 81-84 or of any one of items 81-88, wherein the infusion device comprises a tethered infusion system comprising a reservoir configured to hold the insulin formulation.

90. The system of item 89 or of any one of items 81-89, wherein at least a portion of the electrode arrangement is located at an outlet of the reservoir.

91. The system of item 89 or 90 or of any one of items 81-90, wherein the tethered infusion system comprises an infusion tubing fluidically coupled to the reservoir, wherein at least a portion of the electrode arrangement is located inline with the infusion tubing.

92. The system of any one of items 89-91 or of any one of items 81-91, wherein the tethered infusion system comprises an infusion hub fluidically coupled to the reservoir, wherein at least a portion of the electrode arrangement is located in the infusion hub.

93. The system of any one of items 89-92 or of any one of items 81-92, wherein at least a portion of the electrode arrangement is located in a body of the reservoir.

94. The system of any one of items 89-93 or of any one of items 81-93, wherein at least a portion of the electrode arrangement is located in a stopper of the reservoir.

95. The system of any one of items 89-94 or of any one of items 81-94, wherein the infusion device comprises an infusion pen comprising a reservoir configured to hold the insulin formulation.

96. The system of item 95 or of any one of items 81-95, wherein at least a portion of the electrode arrangement is located in a body of the reservoir.

97. The system of item 95 or 96 or of any one of items 81-96, wherein at least a portion of the electrode arrangement is located in a stopper of the reservoir.

**Claims**

1. A method for identifying concentration of an analyte, the method comprising:

   generating an input signal having a first set of frequency components across a plurality of frequencies;
   exciting an electrode arrangement with the input signal, wherein the electrode arrangement is in contact with the analyte;
   receiving an output signal from the excited electrode arrangement, the output signal having a second set of frequency components across the plurality of frequencies;
   determining an impedance signature of the analyte across the plurality of frequencies, based on the first and second sets of frequency components; and
   determining a concentration of the analyte based on the impedance signature.

2. The method of claim 1, wherein the input signal is a non-sinusoidal wave.

3. The method of claim 1 or claim 2, wherein the input signal has an operating excitation voltage range of +1.23 V to -1.23 V.

4. The method of any one of claims 1-3, wherein determining the impedance signature of the analyte comprises:

   decomposing the input signal into the first set of frequency components;
   decomposing the output signal into the second set of frequency components; and
   determining an impedance of the analyte at each of the plurality of frequencies based on a comparison of the first and second sets of frequency components.

5. The method of any one of claims 1-4, wherein determining the concentration of the analyte comprises determining compositional information of the analyte.

6. The method of any one of claims 1-5, wherein the electrode arrangement is in an analyte delivery device.

7. The method of any one of claims 1-6, further comprising controlling delivery of the analyte to a user, based at least in part on the determined concentration of the analyte.

8. The method of claim 7, wherein controlling delivery of the analyte comprises:

   determining a volume of fluid including the analyte to be delivered to the user; and
   delivering the determined volume of fluid to the user.

9. The method of claim 7 or 8, wherein controlling delivery of the analyte comprises providing an alert communicating an indication of the determined concentration of the analyte.

10. A system for measuring concentration of an analyte, the system comprising:

    an electrode arrangement in contact with a fluid including the analyte;
    a processor; and
    a memory device operably coupled to the processor and storing instructions that, when executed by the processor, cause the system to perform operations comprising:

    generating an input signal having a first set of frequency components across a plurality of frequencies;
    exciting the electrode arrangement with the input signal;
    receiving an output signal from the excited electrode arrangement, the output signal having a second set of frequency components across the plurality of frequencies;
    determining an impedance signature of the analyte across the plurality of frequencies, based on the first and second sets of frequency components; and

determining a concentration of the analyte based on the impedance signature.

11. The system of claim 10, wherein the input signal is a non-sinusoidal wave.

12. The system of claim 10 or 11, wherein determining the impedance signature of the analyte comprises:

decomposing the input signal into the first set of frequency components;
decomposing the output signal into the second set of frequency components; and
determining an impedance of the analyte at each of the plurality of frequencies based on a comparison of the first and second sets of frequency components.

13. The system of any one of claims 10-12, wherein the system comprises an analyte delivery device.

14. The system of claim 13, wherein the analyte delivery device comprises an insulin pump.

15. The system of any one of claims 10-14, wherein the system comprises an analyte monitoring device.

**FIG. 1A**

**FIG. 1B**

200

210 — generating an input signal having a first set of frequency components across a plurality of frequencies

220 — exciting an electrode arrangement with the input signal

230 — receiving an output signal from the excited electrode arrangement, the output signal having a second set of frequency components across the plurality of frequencies

240 — determining an impedance signature of the substance across the plurality of frequencies, based on the first and second sets of frequency components

250 — characterizing the substance based on the impedance signature

**FIG. 2**

300

**FIG. 3**

**FIG. 4**

500

510 — generating an input signal having a first set of frequency components across a plurality of frequencies

520 — exciting an electrode arrangement with the input signal

530 — receiving an output signal from the excited electrode arrangement, the output signal having a second set of frequency components across the plurality of frequencies

540 — determining an impedance signature of an analyte across the plurality of frequencies, based on the first and second sets of frequency components

550 — determining a concentration of the analyte based on the impedance signature

**FIG. 5**

Raw ADC Data from Arduino Test Apparatus

Time-Domain Raw Data
(All Insulin Formulations, N=10 per Formulation)

Legend:
- ○ U100 Humalog
- ▷ U100 Novolog
- ◁ U100 Admelog
- ✶ U100 Truvelog
- × U100 Lyumjev
- ▽ U100 Fiasp
- □ U100 Humalog Diluent
- △ U200 Humalog
- ◇ U500 Humilin (expired)

X-axis: Time Index
Y-axis: ADC Raw Value

FIG. 6

**FIG. 7**

EP 4 656 132 A1

FIG. 8

FIG. 9

**FIG. 10**

EP 4 656 132 A1

FIG. 11

**FIG. 12**

**FIG. 13**

**FIG. 14**

FIG. 15

**FIG. 16A**

**FIG. 16B**

**FIG. 17A**

**FIG. 17B**

Europäisches
Patentamt

European
Patent Office

Office européen
des brevets

# EUROPEAN SEARCH REPORT

**Application Number**

EP 25 17 9085

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/305499 A1 (MATSIEV LEONID [US] ET AL) 2 December 2010 (2010-12-02) * paragraphs [0058] - [0060], [0062], [0064], [0101], [0103] - [0106], [0109], [0132]; figures 1a-1c, 2, 3A-3c, 4 * | 1-15 | INV.<br>A61B5/0537<br>A61B5/145<br>A61B5/1468<br>A61B5/00<br>A61M5/14<br>G16H20/17 |
| X | US 2015/196710 A1 (BENNETT JAMES W [US] ET AL) 16 July 2015 (2015-07-16) * paragraphs [0014] - [0016], [0023] - [0026], [0090] - [0094] * | 1-15 | |

TECHNICAL FIELDS
SEARCHED      (IPC)

A61B
G01N
G16H
A61M

The present search report has been drawn up for all claims

| Place of search | Date of completion of the search | Examiner |
|---|---|---|
| The Hague | 30 June 2025 | Faymann, Juan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 ..................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 17 9085

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2025

| Patent document cited in search report | Publication date | Patent family member(s) | | Publication date |
|---|---|---|---|---|
| US 2010305499 A1 | 02-12-2010 | AU | 2010258872 A1 | 15-12-2011 |
| | | CA | 2763776 A1 | 16-12-2010 |
| | | CN | 102460137 A | 16-05-2012 |
| | | EP | 2440910 A2 | 18-04-2012 |
| | | EP | 2545851 A2 | 16-01-2013 |
| | | JP | 5615914 B2 | 29-10-2014 |
| | | JP | 2012529656 A | 22-11-2012 |
| | | KR | 20120037455 A | 19-04-2012 |
| | | RU | 2011154222 A | 20-07-2013 |
| | | SG | 176600 A1 | 30-01-2012 |
| | | US | 2010305499 A1 | 02-12-2010 |
| | | WO | 2010144482 A2 | 16-12-2010 |
| | | ZA | 201108790 B | 27-05-2015 |
| US 2015196710 A1 | 16-07-2015 | EP | 2262551 A1 | 22-12-2010 |
| | | US | 2011009817 A1 | 13-01-2011 |
| | | US | 2011060198 A1 | 10-03-2011 |
| | | US | 2014249503 A1 | 04-09-2014 |
| | | US | 2015196710 A1 | 16-07-2015 |
| | | WO | 2009114115 A1 | 17-09-2009 |

EPO FORM P0459

For more details about this annex : see Official Journal of the European Patent Office, No. 12/82

**EP 4 656 132 A1**

**Patent documents cited in the description**

- US 63654411
- US 19206602 B

- US 10159786 B